(19) Europäisches Patentamt
European Patent Office
Office européen des brevets

(11) **EP 4 116 074 B1**

(12) **EUROPÄISCHE PATENTSCHRIFT**

(45) Veröffentlichungstag und Bekanntmachung des Hinweises auf die Patenterteilung:
**30.07.2025 Patentblatt 2025/31**

(21) Anmeldenummer: **22179154.4**

(22) Anmeldetag: **15.06.2022**

(51) Internationale Patentklassifikation (IPC):
**B29D 35/00** (2010.01)    **B29D 35/08** (2010.01)
**A61F 5/14** (2022.01)    **A43B 13/12** (2006.01)
**A43B 13/38** (2006.01)    **B32B 25/04** (2006.01)
**B32B 5/18** (2006.01)    **B32B 27/06** (2006.01)
**B32B 27/30** (2006.01)

(52) Gemeinsame Patentklassifikation (CPC):
**B29D 35/0054; A43B 13/12; A43B 13/386;**
**A61F 5/14; B29D 35/085; B32B 5/18;**
**B32B 25/045; B32B 27/065; B32B 27/302;**
**B32B 27/306;** B32B 2266/0207; B32B 2274/00

(54) **VERFAHREN ZUR HERSTELLUNG EINES GEGENSTANDES, MIT EINEM MEHRERE ELEMENTE AUFWEISENDEN ELASTISCH KOMPRIMIERBAREN VERBUNDKÖRPER**

METHOD FOR MANUFACTURING AN OBJECT WITH AN ELASTICALLY COMPRESSIBLE COMPOSITE BODY HAVING SEVERAL ELEMENTS

PROCÉDÉ DE FABRICATION D'UN OBJET, DOTÉ D'UN CORPS COMPOSITE POUVANT ÊTRE COMPRIMÉ ÉLASTIQUEMENT COMPRENANT UNE PLURALITÉ D'ÉLÉMENTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.07.2021 DE 102021117332**

(43) Veröffentlichungstag der Anmeldung:
**11.01.2023 Patentblatt 2023/02**

(73) Patentinhaber: **Nora Systems GmbH**
**69469 Weinheim (DE)**

(72) Erfinder:
• **Schmitt, Jürgen**
**64653 Lorsch (DE)**
• **Raffel, Stefan**
**69469 Weinheim (DE)**
• **Kröger, Mario**
**76646 Bruchsal (DE)**

(74) Vertreter: **Reiser & Partner**
**Patentanwälte mbB**
**Weinheimer Straße 102**
**69469 Weinheim (DE)**

(56) Entgegenhaltungen:
EP-A1- 3 267 817    EP-A1- 3 429 386
EP-A1- 3 645 281    US-A- 5 273 702
US-A1- 2002 144 431    US-A1- 2017 297 287

**Beschreibung**

Die Erfindung betrifft ein Verfahren zur Herstellung eines Gegenstandes, insbesondere eines orthopädischen Gegenstands, wie einer Schuheinlage, mit einem mehrere Elemente aufweisenden elastisch komprimierbaren Verbundkörper. Die Erfindung betrifft darüber hinaus eine Kombination von Gegenständen, die einen ersten Körper aus einem ersten expandierten Material und einen zweiten Körper aus einem zweiten Material umfasst.

Gegenstände mit einem mehrere Elemente aufweisenden elastisch komprimierbaren Verbundkörper sind bekannt. Sie werden beispielsweise für orthopädische Gegenstände, wie insbesondere Schuheinlagen verwendet. Dabei ist es bekannt, Schuheinlagen aus mehreren Schichten herzustellen. Die Schichten können insbesondere unterschiedliche Härten und Dichten aufweisen. Auf diese Weise können die Schuheinlagen an unterschiedliche Anforderungen angepasst werden. Insbesondere kann eine weichere Schicht verwendet werden, um eine gute Druckverteilung zum Fuß hin zu erreichen, während mit einer härteren Schicht eine Stabilisierung erreicht wird. Hierzu können die Schichten als Fußbett vorgeformt sein. Die Schichten können zudem an das Gewicht des Benutzers, wie auch die vorgesehene Verwendung der Schuhe (Sportzwecke, Verwendung auf der Straße, im Wald etc.), wie auch Fehlstellungen des Fußes angepasst werden. Besonders wichtig sind derartige Schuheinlagen auch für Personen mit gesundheitlichen Einschränkungen, wie insbesondere Diabetes, da hier eine gute Druckverteilung wichtig ist, um Durchblutungsstörungen im Fuß zu vermeiden.

Aus der DE 10 2004 014 609 A1 ist eine Verbundplatte aus vernetzten geschäumten Kunststoffen bekannt, bei der die Schichten eine unterschiedliche Härte aufweisen. 15.06.2022

Die Verbundplatte wird dadurch hergestellt, dass zwei Schichten des nicht expandierten Kunststoffmaterials bereitgestellt und gemeinsam in einer Presse erhitzt werden. Dabei werden die Schichten aufgeschäumt, zusammen vulkanisiert und dadurch ohne Klebemittel miteinander verbunden. Nachteilig hierbei ist, dass eine Vielzahl von Kombinationen bereitgestellt werden muss, um eine individuelle Anpassung der Schuheinlage zu ermöglichen.

Weiterhin ist es denkbar, expandierte Körper aus vernetztem Kunststoff einzeln zur Verfügung zu stellen. Die Körper müssen dann jedoch von dem Verarbeiter zusammengesetzt und mit einem Klebstoff verbunden werden.

Die EP 3 267 817 A1 beschreibt ein Verfahren zur Herstellung einer Sohlenanordnung. Das beschriebene Verfahren sieht vor, dass unter Verwendung von Pellets und einem weiteren Material zunächst in einer ersten Form ein sohlenförmiger Vorformling hergestellt wird. Dieser kann EVA enthalten. Der Vorformling wird dann zusammen mit einem zweiten Vorformling in eine zweite Form gegeben, in der die Vorformlinge durch Anschmelzen verbunden werden, bevor die Materialien expandiert werden.

Die Erfindung stellt sich die Aufgabe, ein Verfahren anzugeben, das die Herstellung eines Gegenstandes mit einem mehrere Elemente aufweisenden elastisch komprimierbaren Körper vereinfacht. Die Erfindung stellt sich zudem die Aufgabe eine Kombination von Gegenständen anzugeben, welche die einfache Herstellung eines mehrere Elemente aufweisenden elastisch komprimierbaren Verbundkörpers ermöglicht.

Die Aufgabe wird gelöst durch ein Verfahren zur Herstellung eines Gegenstandes insbesondere eines orthopädischen Gegenstands, nach Anspruch 1.

Hierdurch wird die Herstellung des Gegenstandes mit dem mehrere Elemente aufweisenden Verbundkörper erheblich vereinfacht. Insbesondere können der erste und der zweite Körper miteinander verbunden werden, ohne dass hierfür ein Klebstoff oder eine Klebeschicht aufgebracht werden muss. Vielmehr kann eine dauernde Haftung zwischen dem ersten und zweiten Körper bereits dadurch erreicht werden, dass die erste Kontaktfläche des ersten Körpers mit der zweiten Kontaktfläche des zweiten Körpers nach Erwärmung der Körper in Kontakt gebracht und für eine Zeitspanne in Kontakt gehalten werden. Dies kann beispielsweise in einer üblichen in der Orthopädietechnik verwendeten Presse, wie insbesondere in einem Tiefziehgerät, erfolgen. Dies ist deshalb möglich, da aufgrund der Teilvernetzung des ersten und der Nichtvernetzung oder Teilvernetzung des zweiten Materials eine thermoplastische Bindung zwischen dem ersten und dem zweiten Material erreicht werden kann. Vernetzung bezeichnet in der Chemie Reaktionen, bei denen eine Vielzahl einzelner Makromoleküle zu einem dreidimensionalen Netzwerk verknüpft wird. Hierbei werden intermolekulare Vernetzungsbrücken gebildet. Teilvernetzt bedeutet in dem Zusammenhang, dass eine Vernetzung nicht bis zur vollständigen Vernetzung erfolgt. Im Ergebnis kann der Verarbeiter, wie insbesondere eine Orthopädiewerkstätte oder ein Schuhmacher, Körper mit verschiedenen physikalischen Eigenschaften besonders einfach vorhalten und je nach Bedarf miteinander kombinieren. Das trägt zu einer erheblichen Vereinfachung bei, da mit einer geringen Anzahl von Körpern mit unterschiedlichen physikalischen Eigenschaften eine Vielzahl von Kombinationen hergestellt werden kann. Die Herstellung ist dabei besonders sicher, da die erfindungsgemäßen Gegenstände eine zuverlässige und dauerhafte Verbindung ohne die Zugabe von Klebstoff ermöglichen. Insbesondere lässt sich eine hohe Festigkeit der Verbindung zwischen den Elementen erreichen. Das Risiko der Verwendung eines ungeeigneten Klebers wird ausgeschlossen. Zudem ist die Herstellung besonders schnell und einfach, da die Zugabe eines Klebstoffes entfallen kann. Das trägt zudem zu einer Kostenersparnis bei. Mit dem beanspruchten Verfahren lassen sich somit eine Vielzahl von orthopädischen Materialien herstellen, die neben polsternden beispielsweise auch stützende Eigenschaften aufweisen. Aufgrund der Verwendung von expandierten Materialien werden Körper erhalten, die Poren aufweisen und daher besonders gut komprimierbar (vorzugsweise elastisch komprimierbar) sind. Damit lassen sich insbesondere bettende, polsternde und stabilisierende Eigenschaften einer Schuheinlage herstellen. Die Schuheinlage hat dabei eine gute Haltbarkeit und behält ihre elastischen Eigenschaften über lange Zeit bei. Hierzu trägt auch die teilweise Vernetzung des expandierten ersten Materials bei.

Die oben genannte Aufgabe wird gelöst zudem durch eine Kombination von Gegenständen nach Anspruch 14.

Eine solche Kombination von Gegenständen kann insbesondere als ein polsternder orthopädischer Gegenstand, wie vorzugsweise ein Schuhbestandteil ausgebildet sein bzw. zu dessen Herstellung verwendet werden. Vorzugsweise ist der Schuhbestandteil eine Schuheinlage oder eine Schuhinnensohle.

Nachfolgend werden weitere Merkmale der Erfindung beschrieben. Die Merkmale beziehen sich dabei jeweils auf das erfindungsgemäße Verfahren wie auch die erfindungsgemäße Kombination, sofern dies nicht ausdrücklich anders angegeben wird.

Das zweite Material kann erfindungsgemäß ein expandiertes oder ein nicht expandiertes Material sein. Beide Varianten weisen Vorzüge im Hinblick auf die Anpassung der elastischen Eigenschaften wie auch der Stützeigenschaften des Gegenstandes auf. Wenn auch das zweite Material expandiert ist, können besonders gute Polstereigenschaften und gleichzeitig eine gute Verbindbarkeit erreicht werden.

Eine bevorzugte Ausgestaltung der Erfindung sieht vor, dass das zweite Material expandiert ist, wobei die vernetzbare Komponente des zweiten Körpers ein vernetzbares Polymer aufweist, das in dem zweiten Körper in teilvernetzter Form vorliegt. Das trägt zu einer guten Stabilität sowie einer sicheren Verbindung des ersten und zweiten Körpers bei.

Vorzugsweise sind das erste und das zweite Material thermoplastische Materialien.

Die Vernetzungsreaktion, die zur Teilvernetzung führt, kann auf unterschiedliche Weise erreicht werden. Erfindungsgemäß ist dabei bevorzugt, dass die Teilvernetzung im Rahmen einer chemischen Vernetzung erfolgt. Eine Vernetzungsreaktion, die zur Teilvernetzung führt, kann aber auch durch Strahlenvernetzung erreicht werden.

Erfindungsgemäß ist bevorzugt, dass die vernetzbare Komponente des ersten und/oder des zweiten Materials wenigstens eine Verbindung ausgewählt aus einer Gruppe bestehend aus einem peroxidisch vernetzbaren thermoplastischen Polymer und einem peroxidisch vernetzbaren thermoplastischen Elastomer umfasst. Vorzugsweise enthält die vernetzbare Komponente jedenfalls ein peroxidisch vernetzbares thermoplastisches Polymer. Besonders bevorzugt ist, wenn die vernetzbare Komponente des ersten und/oder des zweiten Materials ein peroxidisch vernetzbares thermoplastisches Polymer und ein peroxidisch vernetzbares thermoplastisches Elastomer umfasst. Die vorgenannten Merkmale ermöglichen die Herstellung eines mehrere Elemente aufweisenden elastisch komprimierbaren Gegenstand mit vorteilhaften polsternden und stabilisierenden Eigenschaften.

Eine besonders bevorzugte Ausgestaltung der Erfindung sieht vor, dass die vernetzbare Komponente des ersten und/oder des zweiten Materials wenigstens eine Verbindung ausgewählt aus einer Gruppe bestehend aus Ethylen-Butylacrylat-Copolymer (EBA), Ethylen-Vinylacetat-Copolymer (EVA), funktionalisiertes Ethylen-Butylacrylat-Copolymer, funktionalisiertes Ethylen-Vinylacetat-Copolymer, Ethylen-Ethylacrylat-Copolymer, Ethylen-Methylacrylat-Copolymer, Ethylen-(Meth)acrylsäureCopolymer, Ethylen-2-Ethylhexyacrylat-Colpolymer, Ethylen-Acrylester-Copolymer, Polyolefin (wie beispielsweise Polyethylen (PE) oder Polypropylen (PP) unter anderem in folgenden Varianten: Polyethylen mit sehr geringer Dichte (VLDPE), Lineares Polyethylen mit geringer Dichte (LLDPE), Polyethylen mit geringer Dichte (LDPE), Polyolefin-Elastomere (z. B. POE) und Ethylen-Propylen-Copolymer (EPM)), Styrol-Butadien-Blockcopolymer (SBS), Styrol-Isopren-Blockcopolymer (SIS), Styrol-Isopren-Butadien-Blockcopolymer (SIBS), Styrol-Ethylen-Butadien-Blockcopolymer (SEBS),Styrol-Ethylen-Propylen-Blockcopolymer (SEPS), Hoch-Styrol-Butadien-Copolymer (HSBR - vorzugsweise mit einem Styrolanteil >50%), Thermoplastisches Polyurethan (TPU) umfasst. Polyethylen mit geringer Dichte weist eine Dichte zwischen $0,915\,g/cm^3$ und $0,935\,g/cm^3$ auf. Polyethylen mit sehr geringer Dichte weist eine Dichte zwischen $0,89\,g/cm^3$ und $0,915\,g/cm^3$ auf. Lineares Polyethylen mit geringer Dichte weist eine Dichte zwischen $0,915\,g/cm^3$ und $0,930\,g/cm^3$ auf.

Besonders bevorzugt ist dabei erfindungsgemäß, wenn die vernetzbare Komponente des ersten und/oder des zweiten Materials Ethylen-Vinylacetat-Copolymer (EVA) umfasst. Weiterhin ist bevorzugt, wenn die die vernetzbare Komponente des ersten und/oder des zweiten Materials Lineares Polyethylen geringer Dichte (LLDPE) und/oder Polyethylen mit geringer Dichte (LDPE) umfasst. Zudem ist bevorzugt, wenn die die vernetzbare Komponente des ersten und/oder des zweiten Materials Ethylen-Vinylacetat-Copolymer (EVA) und Hoch-Styrol-Butadien-Copolymer (HSBR) (mit Styrolanteil >50%) umfasst. Außerdem ist bevorzugt, wenn die die vernetzbare Komponente des ersten und/oder des zweiten Materials wenigstens eine der Verbindungen Ethylen-Methylacrylat-Copolymer, Ethylen-Ethylacrylat-Copolymer, Ethylen-Butylacrylat-Copolymer und Ethylen-(Meth)acrylsäure-Copolymer umfasst.

Eine weitere bevorzugte Ausgestaltung der Erfindung sieht vor, dass die vernetzbare Komponente zusätzlich ein styrolhaltiges Polymer umfasst. Dies ermöglicht die Herstellung eines mehrere Elemente aufweisenden elastisch komprimierbaren Gegenstand mit vorteilhaften polsternden und stabilisierenden Eigenschaften. Dabei kann vorgesehen sein, dass die vernetzbare Komponente zusätzlich zu dem styrolhaltigen Polymer auch ein nicht styrolhaltiges Polymer aufweist.

Erfindungsgemäß ist besonders bevorzugt, wenn das styrolhaltige Polymer wenigstens eine Verbindung ausgewählt aus der Gruppe bestehend aus Styrol-Butadien-Blockcopolymer (SBS), Styrol-Isopren-Blockcopolymer (SIS), Styrol-Isopren-Butadien-Blockcopolymer (SIBS), Styrol-Ethylen-Butadien-Blockcopolymer (SEBS), Styrol-Ethylen-Propylen-Blockcopolymer (SEPS), Hoch-Styrol-Butadien-Copolymer (HSBR, vorzugsweise mit einem Styrolanteil >50%) umfasst. Besonders bevorzugt ist dabei erfindungsgemäß, wenn das styrolhaltige Polymer Styrol-Butadien-Blockcopolymer und/oder Styrol-Isopren-Blockcopolymer umfasst.

Eine bevorzugte Ausgestaltung sieht vor, dass der Styrolgehalt des styrolhaltigen Polymers bei mehr als 50% liegt. Vorzugweise liegt der Styrolgehalt bei mehr als 60 %. Weiterhin ist bevorzugt, wenn der Styrolgehalt des styrolhaltigen Polymers weniger als 80 % beträgt.

Eine weitere Verbesserung der Eigenschaften des mehrere Elemente aufweisenden komprimierbaren Gegenstands wird dadurch erreicht, dass wenigstens eines des ersten und des zweiten Materials (vorzugsweise beide) zusätzlich wenigstens ein vernetzbares Elastomer aufweist, das in dem ersten bzw. zweiten Material in teilvernetzter Form vorliegt. Dies ermöglicht die Herstellung eines mehrere Elemente aufweisenden elastisch komprimierbaren Gegenstand mit vorteilhaften polsternden und stabilisierenden Eigenschaften. Insbesondere kann das vernetzbare Elastomer eine Kautschukverbindung umfassen. Vorzugsweise ist das vernetzbare Polymer peroxidisch vernetzbar.

Erfindungsgemäß ist dabei bevorzugt, dass das peroxidisch vernetzbare Elastomer wenigstens eine Verbindung ausgewählt aus der Gruppe bestehend aus Styrol-Butadien-Kautschuk (SBR), Nitril-Butadien-Kautschuk (NBR), Ethylen-Propylen-Dien-Kautschuk (EPDM), Naturkautschuk (NR), Isopren-Kautschuk (IR), Butadien-Kautschuk (BR), Polyacrylat-Kautschuk (ACM) und Polyethylen-Acrylat-Kautschuk (AEM) umfasst. Besonders bevorzugt sind Nitril-Butadien-Kautschuk (NBR), Isopren-Kautschuk (IR) und Naturkautschuk (NR) sowie Mischungen hiervon.

Eine weitere Verbesserung sieht vor, dass die vernetzbare Komponente des ersten Körpers und die vernetzbare Komponente des zweiten Körpers wenigstens ein übereinstimmendes vernetzbares Polymer aufweisen.

Eine bevorzugte Ausgestaltung der Erfindung sieht vor, dass das erste und/oder das zweite expandierte Material einen Füllstoff aufweist. Mit dem Füllstoff kann die Härte wie auch die Dichte des ersten und zweiten expandierten Materials eingestellt werden. Zudem trägt der Füllstoff zu einer hohen Stabilität des ersten und zweiten Materials bei. Auf diese Weise kann mit dem Füllstoff auch die Festigkeit der Verbindung zwischen dem ersten und dem zweiten Körper verbessert werden.

In bevorzugter Weise umfasst der Füllstoff wenigstens eine Verbindung ausgewählt aus einer Gruppe bestehend aus Kreide, Ruß, Kieselerde, Kieselsäure, Kaolin, Aluminiumhydroxid, Natrium-Aluminium-Silikat, Glasmehl, Holzmehl, Nussschalenmehl, Lignin und Zellulose.

Vorzugsweise wird dem Basismaterial dabei ein Füllstoff zugegeben.

Der zweite Körper kann in bevorzugter Weise entsprechend hergestellt werden. Dementsprechend kann vorgesehen sein, dass der zweite Körper dadurch hergestellt wird, dass zunächst ein zweites Basismaterial hergestellt wird, indem die vernetzbare Komponente in unvernetzter Form, ein chemisches Treibmittel und ein Vernetzungsmittel zusammen gemischt werden, dass das chemische Treibmittel und das Vernetzungsmittel dann (vorzugsweise unter Zufuhr von Energie) aktiviert werden, sodass das Basismaterial expandiert und die vernetzbare Komponente teilvernetzt wird, um das expandierte Material des zweiten Körpers mit teilvernetzten Polymeren zu erhalten. Vorzugsweise wird dem Basismaterial dabei ein Füllstoff zugegeben.

Andererseits ist es jedoch auch möglich, den zweiten Körper ohne Treibmittel in dem zweiten Basismaterial herzustellen. Der erhaltene zweite Körper ist dann nicht expandiert. Hierbei kann ein Vernetzungsmittel in dem ersten Basismaterial enthalten sein, wenn die Polymere des zweiten Körpers teilvernetzt werden sollen. Die Herstellung ist jedoch auch ohne Vernetzungsmittel möglich. Die Polymere des zweiten Basismaterial bleiben dann unvernetzt.

Das Mischen kann dabei jeweils in einem Mischaggregat, wie insbesondere einem Innenmischer (z. B. Kneter) erfolgen. Die Aktivierung des Treibmittels und des Vernetzungsmittels erfolgt vorzugsweise in einer Form, beispielsweise in der Form einer Heizpresse.

Das erste Basismaterial und gegebenenfalls das zweite Basismaterial erhalten vorzugsweise mindestens ein chemisches Treibmittel. Unter einem chemischen Treibmittel werden Verbindungen verstanden, die sich bei Einwirkung von Wärme zersetzen und dabei Gase freisetzen.

Erfindungsgemäße Beispiele für geeignete chemische Treibmittel sind Azoverbindungen, Hydrazidverbindungen, Nitrosoverbindungen und Carbazidverbindungen, wie beispielsweise Azobisisobutyronitril, Azodicarbonamid (ADCA), Di-nitroso-pentamethylentetramin, 4,4'-Oxybis(benzolsulfonsäure hydrazid) (OBSH), Azocyclohexylnitril, Azodiaminobenzol, Benzol-1,3-sulfonylhydrazid, Calciumazid, 4,4'-Diphenyldisulfonylazid, Diphenyl-sulfon-3,3'-disulfohydrazid, Benzol-1,3-disulfohydrazid, Trihydrazinotriazin, p-Toluolsulfonylhydrazid und p-Toluolsulfonylsemicarbazid.

Im Rahmen dieser Ausführungsform haben sich Zubereitungen als vorteilhaft erwiesen, die einen Gehalt von Azodicarbonamid von 0,5 bis 8 Gew.-%, insbesondere von 2 bis 5 Gew.-%, jeweils bezogen auf die Gesamtmasse des ersten bzw. zweiten Basismaterials enthalten.

Weiterhin ist bevorzugt, dass das erste und/oder das zweite Basismaterial ein chemisches Vernetzungsmittel aufweisen. Dabei kann vorgesehen sein, dass das Vernetzungsmittel in dem ersten bzw. dem zweiten Basismaterial jeweils in einer Menge enthalten ist, welche eine teilweise Vernetzung der vernetzbaren Komponente ermöglicht, jedoch für eine vollständige Vernetzung nicht ausreicht.

Vorzugsweise umfasst das chemische Vernetzungsmittel ein Peroxid.

Erfindungsgemäß sind insbesondere die organischen Peroxide, wie beispielsweise Ketonperoxide, Diacylperoxide, Perester, Perketale und Hydroperoxide bevorzugt. Besonders bevorzugt sind beispielsweise Cumenhydroperoxid, t-Butylperoxid, Bis(tert-butylperoxy)-diisopropylbenzol, Di(tert-butylperoxyisopropyl)benzol, Dicumylperoxid, t-Butylperoxybenzoat, Di-alkylperoxydicarbonat, Diperoxyketale (z. B. 1,1-Di-tert-butylperoxy-3,3,5-trimethylcyclohexan), Ketonperoxide (z. B. Methylethylketon-Peroxide) und 4,4- Di-tert-butylperoxy-n-butyl-valerate. Besonders bevorzugt sind erfindungsgemäß die beispielsweise kommerziell von der Firma Akzo Nobel vertriebenen Peroxide, wie 3,3,5,7,7-Pentamethyl-1,2,4-trioxepan, 2,5-Dimethyl-2,5-di(tert-butylperoxy)hex-3-in, Di-tert-butylperoxid, 2,5-Dimethyl-2,5-di(tert-butylperoxy)hexan, tert-Butylcumylperoxid, Di(tert-butylperoxyisopropyl)benzol, Dicumylperoxid, Butyl-4,4-di(tert-butylperoxi)valerat, tert-Butylperoxy-2-ethylhexylcarbonat, 1,1-Di-(tert-butylperoxy)-3,3,5-trimethylcyclohexan, tert-Butylperoxybenzoat, Di-(4-methylbenzoyl)peroxid und Dibenzoylperoxid.

Es hat sich weiterhin als erfindungsgemäß vorteilhaft erwiesen, wenn die eingesetzten Peroxide bei Raumtemperatur im Wesentlichen inert sind und erst bei Erhitzung auf höhere Temperaturen aktiviert werden (beispielsweise bei Erhitzung auf Temperaturen zwischen 130°C und 240°C). Besonders vorteilhaft ist es erfindungsgemäß, wenn das eingesetzte Peroxid bei 65°C eine Halbwertszeit von mehr als 60 Minuten aufweist, das heißt, dass nach einer Erhitzung des ersten bzw. zweiten Basismaterials enthaltend das Peroxid auf 65°C für 60 Minuten sich weniger als die Hälfte des eingesetzten Peroxids zersetzt hat. Erfindungsgemäß können solche Peroxide besonders bevorzugt sein, die bei 115°C eine Halbwertszeit von 60 Minuten aufweisen.

Es kann erfindungsgemäß besonders bevorzugt sein, Di(tert-butylperoxyisopropyl)benzol als Peroxid einzusetzen; dies ist beispielsweise unter den Handelsbezeichnungen Perkadox® 14-40 B-PD oder Perkadox® 14-40 K PD von der Firma Akzo Nobel oder unter den Handelsbezeichnung Peroxan® BIB 40 GS oder Peroxan® BIB 40 P von der Firma Pergan kommerziell erhältlich.

In einer weiteren erfindungsgemäßen Form kann es ebenso bevorzugt sein, Dicumylperoxid, wie es beispielsweise unter den Handelsbezeichnungen Perkadox® BC 40 K PD oder Perkadox® BC 40 B PD von der Firma Akzo Nobel oder unter den Handelsbezeichnungen Peroxan® DC 40 GS, Peroxan® DC 40 P oder Peroxan® DC 40 PK von der Firma Pergan vertrieben wird, einzusetzen.

Das Vernetzungsmittel ist in dem ersten bzw. dem zweiten Basismaterial zur Erreichung der angestrebten Teilvernetzung vorzugsweise in einer Menge von 0,05 bis 0,5 Gew.-%, insbesondere in einer Menge von 0,2 bis 0,4 Gew.-%, jeweils bestimmt als Aktivsubstanzgehalt an Peroxid bezogen auf die Masse des ersten bzw. zweiten Basismaterials, enthalten.

Eine besonders bevorzugte Ausgestaltung der Erfindung sieht vor, dass der Vernetzungsgrad des ersten Körpers und/oder des zweiten Körpers unter 92 % liegt. Besonders gute Eigenschaften werden dann erzielt, wenn der Vernetzungsgrad unter 90 % liegt. Vorzugsweise liegt der Vernetzungsgrad des ersten und/oder zweiten Körpers über 25%. Dabei ist bevorzugt, wenn der Vernetzungsgrad über 50% liegt, wobei ein Vernetzungsgrad von mehr als 60% besonders bevorzugt ist.

In bevorzugter Weise kann der vorgenannte Vernetzungsgrad analog zu DIN 16892:2000-07 bestimmt werden. Die DIN 16892:2000-07 bezieht sich auf Rohre aus vernetztem Polyethylen hoher Dichte. Die Norm beschreibt ein Vorgehen zur Ermittlung des Vernetzungsgrads, welches auch bei dem beanspruchten ersten und zweiten Material zur Anwendung gebracht werden kann. Das Verfahren basiert darauf, den Vernetzungsgrad dadurch zu bestimmen, dass die in siedendem Xylol unlöslichen Massenanteile des Materials bestimmt werden.

Das Verfahren sieht Folgendes vor:

Probenentnahme: Von dem Material werden Proben in Form von kleinen Spänen, z. B. als Scherenspäne, entnommen.

Durchführung: Von der entnommenen Probe (Masse $m_1$) werden 0,5 g (+- 0,1 g) auf 1 mg gewogen und in einen Behälter aus Drahtnetzen oder perforierten Blechen gelegt. Der Behälter mit der Probe wird in siedendem technisch reinem Xylol gelagert. Dem Lösemittel ist 1 % Antioxidationsmittel (2,2-Methylen-bis-(4-methyl-6 tert-butylphenol) bzw. Pentaerythrityltetrakis (3-(3,5 di-tert.butyl- 4-hydroxiphenyl)-propionate)) zugefügt. Die Lagerungsdauer beträgt 8 h ± 5 min. Anschließend wird der Behälter mit dem Rückstand aus dem noch siedenden Lösemittel entnommen, auf Raumtemperatur abgekühlt und getrocknet. Die in Xylol gelagerten Proben werden 3 h, in einem auf 140 °C aufgeheizten, im Frischluftbetrieb arbeitenden Wärmeschrank mit zwangsläufiger Durchlüftung getrocknet. Nach Abkühlung auf Raumtemperatur wird die Masse des Rückstands (Masse $m_2$) auf 1 mg bestimmt, Auswertung: Der in Xylol unlösliche Massenanteil entspricht dem Vernetzungsgrad G in % und wird nach Gleichung (5) berechnet:

$$G = \frac{m_2}{m_1} \cdot 100$$

Auf diese Weise kann der Vernetzungsgrad G in % bestimmt werden.

Da sich bei der Lagerung der Probe in Xylol unter Umständen aus dem untersuchten Material auch andere Inhaltsstoffe als nichtvernetzte Polymere (z. B. enthaltene Verarbeitungshilfsmittel) lösen können, kann auch bei Körpern, die ein vollvernetztes Polymer enthalten, der wie zuvor analog zu der DIN 16892:2000-07 ermittelte Vernetzungsgrad G geringfügig unter 100 % liegen.

Bei der Herstellung eines orthopädischen Gegenstands, wie insbesondere einer Schuheinlage, kann vorgesehen sein, dass der erste und der zweite Körper vor oder nach dem Herstellen des Verbundkörpers zugeschnitten werden.

Bei der Herstellung eines orthopädischen Gegenstands, wie insbesondere einer Schuheinlage, kann weiterhin vorgesehen sein, dass der Verbundkörper nach Erwärmung plastisch verformbar ist. Auf diese Weise kann der Verbundkörper beispielsweise zu einem geformten Fußbett umgeformt werden.

Eine erfindungsgemäße Ausgestaltung sieht vor, dass dem ersten und/oder dem zweiten Basismaterial Pigmente und/oder Verarbeitungshilfsmittel zugegeben werden.

Erfindungsgemäß ist bevorzugt, dass das vernetzbare Elastomer in dem ersten und/oder dem zweiten Basismaterial in einer Menge von jeweils zwischen 5 und 30 Gewichtsanteilen pro 100 Gewichtsanteile Polymer enthalten ist.

Die Angabe 100 Gewichtsanteile Polymer bezieht sich dabei jeweils auf die Gesamtmasse aller Polymere in dem ersten bzw. dem zweiten Basismaterial.

Erfindungsgemäß ist bevorzugt, dass das styrolhaltige Polymer in dem ersten und/oder dem zweiten Basismaterial in einer Menge von jeweils zwischen 5 und 30 Gewichtsanteilen pro 100 Gewichtsanteile Polymer enthalten ist. Erfindungsgemäß ist bevorzugt, dass der Füllstoff in dem ersten und/oder dem zweiten Basismaterial in einer Menge von jeweils zwischen 5 und 50 Gewichtsanteilen pro 100 Gewichtsanteile Polymer enthalten ist. Erfindungsgemäß ist bevorzugt, dass der erste und der zweite Körper unterschiedliche physikalische Eigenschaften aufweisen.

Insbesondere können der erste und der zweite Körper eine unterschiedliche Härte und/oder Dichte aufweisen. Auf diese Weise kann eine weichere Schicht mit guten Polstereigenschaften mit einer härteren Schicht, die eine gute Stabilisierung ermöglicht, kombiniert werden.

Die Shore-Härte kann jeweils entsprechend der DIN ISO 48-4: 2018-08 bestimmt werden.

Erfindungsgemäß ist der erste Körper weicher als der zweite Körper.

Erfindungsgemäß ist vorgesehen, dass der erste Körper eine Härte zwischen 5 und 50 Shore A aufweist.

Erfindungsgemäß ist vorgesehen, dass der zweite Körper eine Härte zwischen 20 und 75 Shore A aufweist.

Erfindungsgemäß ist weiterhin bevorzugt, dass die Dichte des ersten und des zweiten Körpers zwischen 0,05 und 0,7 g/cm$^3$ liegt. In bevorzugter Weise ist die Dichte des ersten Körpers geringer als die Dichte des zweiten Körpers. Bevorzugte Werte für die Dichte des ersten Körpers liegen zwischen 0,08 g/cm$^3$ und 0,3 g/cm$^3$. Bevorzugte Werte für die Dichte des zweiten Körpers liegen zwischen 0,15 g/cm$^3$ und 0,5 g/cm$^3$.

Eine vorteilhafte Ausführungsform sieht vor, dass die Verbindung des ersten und des zweiten Körpers derart ausgebildet ist, dass der Schälwiderstand zwischen 0,5 N/mm und 5 N/mm beträgt. Der Schälwiderstand kann dabei entsprechend DIN EN 1392: 2006-08 ermittelt werden. Vorzugsweise liegt der Schälwiderstand zwischen 1 N/mm und 3 N/mm.

Eine vorteilhafte erfindungsgemäße Variante sieht vor, dass der Verbundkörper ein Schichtkörper ist, wobei der erste Körper eine erste Schicht und der zweite Körper eine zweite Schicht bildet. Vorzugsweise sind die erste und die zweite Schicht übereinander angeordnet. Insbesondere können die erste und die zweite Schicht sandwichartig übereinander angeordnet sein. Vorzugsweise ist die erste Schicht auf der dem Fuß zugewandten Oberseite einer Schuheinlage angeordnet. Auf diese Weise können besonders gute elastische Eigenschaften erreicht werden.

Dabei ist bevorzugt, dass die Schichtstärke der ersten und/oder der zweiten Schicht jeweils zwischen 2 mm und 20 mm liegt. Vorzugsweise liegt die Schichtstärke jeweils zwischen 3 mm und 10 mm.

Erfindungsgemäß sind der erste Körper und der zweite Körper plattenförmig ausgebildet. Aus den Platten können Abschnitte mit einer Größe ausgeschnitten werden, die für die Herstellung einer Schuheinlage geeignet sind.

Eine weitere erfindungsgemäße Gestaltung sieht vor, dass der erste und der zweite Körper in derselben Schicht angeordnet sind, wobei der erste und der zweite Körper jeweils einen Abschnitt der Schicht bilden. Auf diese Weise können abschnittweise unterschiedliche elastische bzw. polsternde Eigenschaften erreicht werden. Gleichzeitig können der erste und der zweite Körper erfindungsgemäß sicher verbunden werden. Hierbei ist es beispielsweise möglich, den ersten Körper neben dem zweiten Körper anzuordnen. Außerdem ist es möglich, dass einer des ersten und zweiten Körpers eine Aussparung aufweist, in der der andere des ersten und zweiten Körpers eingesetzt ist.

Eine bevorzugte Ausgestaltung sieht vor, dass an der durch die erste und die zweite Kontaktfläche gebildeten Fügestelle das erste expandierte Material unmittelbar mit dem zweiten Material thermoplastisch gefügt ist. Insbesondere kann eine Verbindung zwischen dem ersten und dem zweiten expandierten Material bestehen, bei der Haftkräfte unmittelbar zwischen dem ersten und dem zweiten expandierten Material vorliegen. Unmittelbare Verbindung bedeutet in diesem Zusammenhang, dass die Materialien ohne Zwischenschicht und/oder Klebstoff direkt miteinander verbunden sind.

Um eine thermoplastische Verbindung zu erreichen, können beim Herstellen des Verbundkörpers der erste und der zweite Körper auf eine Temperatur zwischen 130°C und 170°C erwärmt werden.

Die Zeitspanne, für die der erste und der zweite Körper miteinander in Kontakt gehalten werden, beträgt vorzugsweise zwischen 4 und 40 Minuten.

Vorzugsweise ist vorgesehen, dass das erste und/oder das zweite expandierte Material offene und/oder geschlossene Poren aufweisen. Dabei ist bevorzugt, wenn das erste und/oder das zweite expandierte Material geschlossenporig ausgebildet sind.

Es liegt auch im Rahmen der Erfindung, dass der elastisch verformbare Körper mehr als zwei Elemente aufweist. Dementsprechend kann vorgesehen sein, dass wenigstens ein weiterer Körper bereitgestellt wird, der wie der erste oder zweite Körper ausgebildet ist und eine dritte Kontaktfläche aufweist. Der erste oder der zweite Körper kann eine vierte Kontaktfläche aufweisen. Der weitere Körper liegt dann mit der dritten Kontaktfläche an der vierten Kontaktfläche an. Eine dauerhafte Verbindung kann wie zwischen dem ersten und dem zweiten Körper ohne Klebstoff erreicht werden. Auf die Beschreibung zu dem ersten und dem zweiten Körper, welche entsprechend für den bzw. die weiteren Körper gilt, wird verwiesen. Mit Hilfe des weiteren Körpers bzw. der weiteren Körper kann beispielsweise ein drei- oder mehrschichtiger Aufbau des komprimierbaren Gegenstands erreicht werden. Dabei kann der weitere Körper (bzw. die weiteren Körper) physikalische Eigenschaften aufweisen, welche von dem ersten und/oder dem zweiten Körper abweichen. Insbesondere kann der weitere Körper eine andere Härte und/oder eine andere Dichte als der erste und/oder der zweite Körper aufweisen. Auf diese Weise werden die Möglichkeiten, eine gute Polsterung und gleichzeitig eine gute Stützung zu erreichen nochmals verbessert. Das ist für orthopädische Anwendungen besonders vorteilhaft.

Erfindungsgemäß kann vorgesehen sein, dass der mehrere Elemente aufweisende elastisch komprimierbare Gegenstand ein orthopädisches Polster- und/oder Aufbaumaterial ist. Der mehrere Elemente aufweisende elastisch komprimierbare Gegenstand kann aber auch für andere technische Anwendungszwecke ausgestaltet sein.

Weitere Ziele, Merkmale, Vorteile und Anwendungsmöglichkeiten der vorliegenden Erfindung ergeben sich aus der nachfolgenden Beschreibung von Ausführungsbeispielen anhand der Zeichnungen. Dabei bilden alle beschriebenen und/oder bildlich dargestellten Merkmale für sich oder in beliebiger sinnvoller Kombination den Gegenstand der Erfindung, auch unabhängig von der Zusammenfassung in einzelnen Ansprüchen oder deren Rückbeziehungen.

Es zeigen:

Fig. 1:     einen erfindungsgemäßen Gegenstand mit einem zwei Elemente aufweisenden elastisch komprimierbaren Verbundkörper;

Fig. 2:     eine erfindungsgemäße Schuheinlage, mit einem zwei Elemente aufweisenden elastisch komprimierbaren Verbundkörper;

Fig. 3:     einen erfindungsgemäßen Gegenstand mit einem drei Elemente aufweisenden elastisch komprimierbaren Verbundkörper und

Fig. 4:     eine weitere erfindungsgemäße Schuheinlage, mit einem drei Elemente aufweisenden elastisch komprimierbaren Verbundkörper;

Fig. 5:     einen weiteren erfindungsgemäßen Gegenstand mit einem zwei Elemente aufweisenden elastisch komprimierbaren Verbundkörper;

Fig. 6a:    eine Seitenansicht eines weiteren erfindungsgemäßen Gegenstands mit einem drei Elemente aufweisenden elastisch komprimierbaren Verbundkörper;

Fig. 6b:    den Gegenstand aus Fig. 6a in Draufsicht.

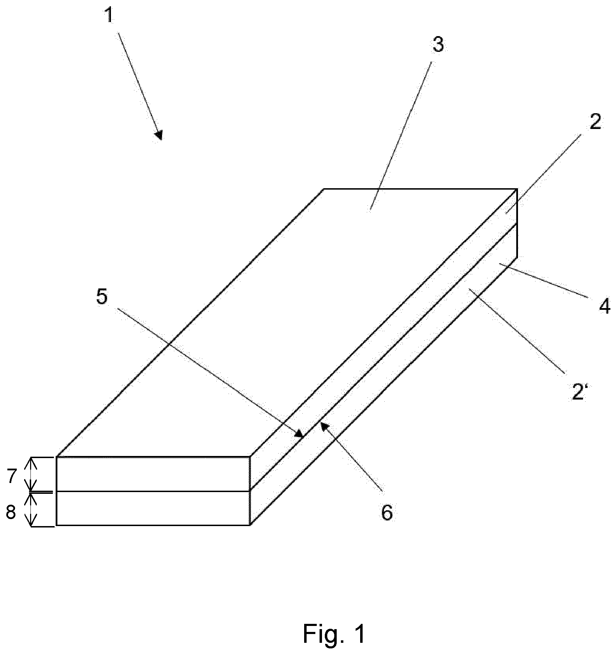

Figur 1 zeigt in perspektivischer Darstellung einen Verbundkörper 1, der mehrere Elemente 2, 2' aufweist. Der Verbundkörper 1 ist elastisch komprimierbar. Er umfasst einen ersten Körper 3 aus einem ersten expandierten Material sowie einen zweiten Körper 4 aus einem zweiten Material, das vorzugsweise ebenfalls expandiert ist. Das erste und das zweite expandierte Material weisen Poren auf und sind elastisch komprimierbar.

Der erste und der zweite Körper 3, 4 bilden jeweils eine Schicht des Verbundkörpers 1.

Der erste Körper 3 weist eine erste Kontaktfläche 5 auf. Der zweite Körper 4 weist eine zweite Kontaktfläche 6 auf. Der erste und der zweite Körper 3, 4 liegen mit der ersten und der zweiten Kontaktfläche 5, 6 aneinander an und sind an dieser dauerhaft miteinander verbunden. Hierzu weisen das expandierte Material des ersten Körpers 3 und das expandierte Material des zweiten Körpers 4 jeweils eine vernetzbare Komponente auf, die in teilvernetzter Form vorliegt. Damit sind der erste Körper und der zweite Körper 3, 4, wie nachfolgend noch genauer beschrieben wird, miteinander an der ersten und der zweiten Kontaktfläche 5, 6 unmittelbar miteinander verbunden. Ein Klebstoff oder eine zusätzliche Verbindungsschicht ist zwischen dem ersten und dem zweiten Körper nicht vorgesehen.

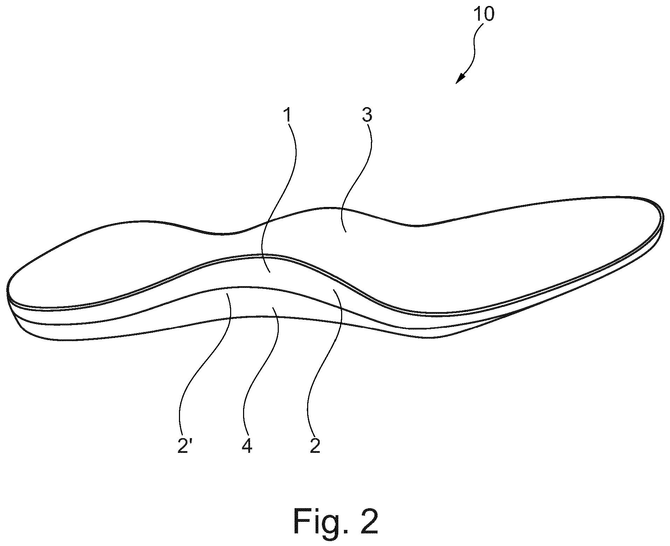

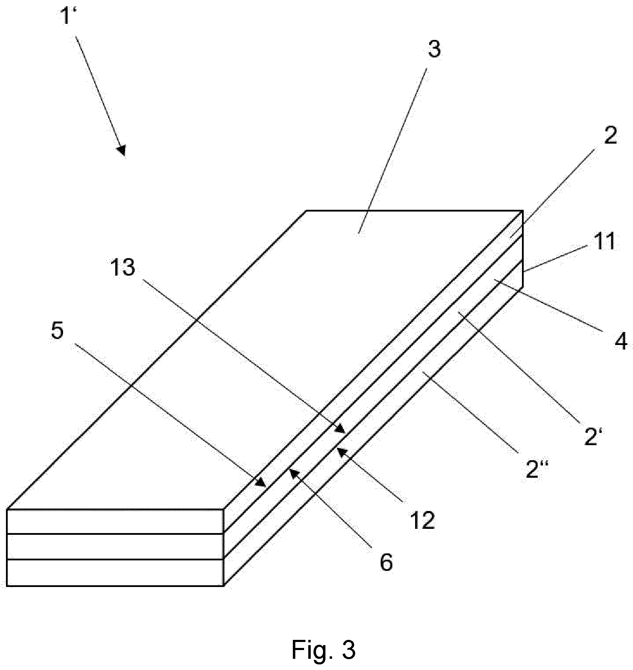

Figur 1 zeigt dabei eine plattenförmige Gestalt des Verbundkörpers 1. Aus dem Verbundkörper 1 können Gegenstände für einen bestimmten Verwendungszweck, wie insbesondere die in den Figuren 2 und 3 dargestellten Schuheinlagen hergestellt werden.

Der erste und der zweite Körper 3, 4 weisen unterschiedliche physikalische Eigenschaften auf. Insbesondere für die Anwendung in dem Bereich der Orthopädie und der Schuheinlagen hat sich bewährt, wenn die Härte des ersten und des zweiten Körpers 3, 4 zwischen 5 Shore A und 75 Shore A beträgt. Dabei kann der erste Körper 3 weicher ausgebildet sein als der zweite Körper 4. Die Härte kann insbesondere durch den Grad der Expansion, die Auswahl der für das erste und das zweite expandierte Material verwendeten Polymere. wie auch die Menge des verwendeten Füllstoffs eingestellt werden. Dabei führt eine stärkere Expansion zu einem größeren Porenvolumen und somit zu einem weicheren Material.

Weiterhin kann vorgesehen sein, dass sich die Dichten des ersten und des zweiten Körpers 3, 4 unterscheiden. Insbesondere kann die Dichte ersten und des zweiten Körpers 3, 4 in dem Bereich zwischen 0,05 g/cm$^3$ und 0,7 g/cm$^3$ liegen. Insbesondere kann der erste Körper 3 eine geringere Dichte als der zweite Körper 4 aufweisen. Dabei ist bevorzugt, wenn die Dichte des ersten Körpers 3 zwischen 0,08 g/cm$^3$ und 0,3 g/cm$^3$ liegt. Für die Dichte des zweiten Körpers 4 sind Werte zwischen 0,15 g/cm$^3$ und 0,5 g/cm$^3$ bevorzugt. Die Dichte des ersten und des zweiten Körpers 3, 4 kann ebenfalls durch den Grad der Expansion des expandierten Materials eingestellt werden. Eine höhere Expansion führt dabei zu einem größeren gesamten Porenvolumen und somit zu einer geringeren Dichte. Die Dichte wird außerdem durch die Menge und die Art des verwendeten Füllstoffs beeinflusst.

Die Schichtstärke 7, 8 des ersten und des zweiten Körpers 3, 4 kann je nach Anwendungszweck unterschiedlich ausgestaltet sein. Bevorzugt liegt die Schichtstärke der durch den ersten und den zweiten Körper 3, 4 gebildeten Elemente 2, 2' jeweils zwischen 2 mm und 20 mm.

Der erste und der zweite Körper 3, 4 sind dauerhaft miteinander verbunden. Die Festigkeit der Verbindung zwischen erstem und zweitem Körper 3, 4 kann durch Ermittlung des Schälwiderstands überprüft werden. Der Schälwiderstand kann entsprechend zu DIN EN 1392: 2006-08 ermittelt werden.

Der erste und der zweite Körper 3, 4 werden wie nachfolgend näher erläutert zunächst separat hergestellt. Um den ersten und den zweiten Körper 3, 4 miteinander zu verbinden, werden der erste und der zweite Körper 3, 4 erwärmt und dann übereinander angeordnet, sodass diese an der ersten und zweiten Kontaktfläche 5, 6 aneinander anliegen. Sodann werden sie für eine Zeitspanne T unter Druck miteinander in Kontakt gehalten und dabei abgekühlt. Das kann insbesondere in einem orthopädischen Tiefziehgerät erfolgen. Dabei ist eine Erwärmung auf Temperaturen zwischen 130 °C und 170 °C geeignet. Die Zeitspanne T liegt bevorzugt zwischen 4 Minuten und 40 Minuten. Dabei ergibt sich eine dauerhafte Verbindung unmittelbar zwischen dem ersten Körper 3 und dem zweiten Körper 4, ohne dass hierfür Klebstoff zugegeben werden müsste.

Figur 2 zeigt eine Schuheinlage 10 mit einem Verbundkörper 1. Die Schuheinlage ist aus einem Verbundkörper 1, wie in Figur 1 dargestellt, hergestellt. Dementsprechend weist die Schuheinlage 10 die schichtartig angeordneten Elemente 2, 2' auf, die durch den ersten und den zweiten Körper 3, 4 gebildet werden.

Figur 3 zeigt einen Gegenstand mit einem drei Elemente 2, 2', 2" aufweisenden Verbundkörper 1'. Die Elemente 2, 2', 2" sind dabei als Schichten angeordnet. Der Verbundkörper 1' weist zusätzlich zu dem ersten Körper 3 und dem zweiten Körper 4, die wie bei dem in Figur 1 dargestellten Ausführungsbeispiel ausgebildet sind, einen weiteren Körper 11 auf. Der weitere Körper 11 weist eine dritte Kontaktfläche 12 auf. Eine vierte Kontaktfläche 13 ist an dem zweiten Körper 4 ausgebildet. Der zweite Körper 4 und der weitere Körper 11 liegen mit der dritten und der vierten Kontaktfläche 12, 13 aneinander an.

Der weitere Körper 11 ist im Wesentlichen wie der erste Körper 3 beziehungsweise der zweite Körper 4 ausgebildet. Die Beschreibung des ersten Körpers 3 und des zweiten Körpers 4 soll daher entsprechend auch für den weiteren Körper 11 gelten. Der weitere Körper 11 kann jedoch andere physikalische Eigenschaften und insbesondere eine andere Härte und/oder Dichte als der erste Körper 3 und/oder der zweite Körper 4 aufweisen. Dabei können die Härte und die Dichte in den für den ersten und zweiten Körper 3, 4 genannten Bereichen liegen. Auf diese Weise können orthopädische Gegenstände, wie insbesondere Schuheinlagen, hergestellt werden, die besonders günstige Eigenschaften haben. Insbesondere lassen sich auf diese Weise elastische und stabilisierende Eigenschaften verbinden und an den jeweiligen Anwendungsfall anpassen.

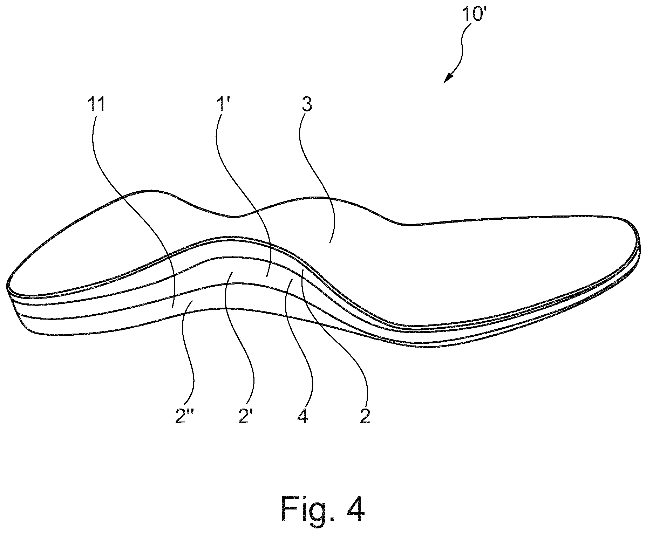

Figur 4 zeigt eine Schuheinlage 10' mit einem Verbundkörper 1'. Die Schuheinlage 10' ist aus einem Verbundkörper 1', wie in Figur 3 dargestellt, hergestellt. Dementsprechend weist die Schuheinlage 10' die Elemente 2, 2' und 2" auf, die durch den ersten, den zweiten und den weiteren Körper 3, 4, 11 gebildet werden.

Die Schuheinlagen 10, 10' können unter Verwendung des Verbundkörpers 1 bzw. 1' hergestellt werden. Hierzu wird aus dem Verbundkörper 1 bzw. 1', wenn er größer als erforderlich ist, ein der Fußgröße entsprechender Abschnitt ausgeschnitten. Der Verbundkörper 1, 1' kann dann einem Formgebungsschritt unterzogen werden, um eine an das Fußgewölbe angepasste Form zu erhalten. Aufgrund der thermoplastischen Eigenschaften des Verbundkörpers 1 beziehungsweise 1' kann durch nochmaliges Erwärmen des Verbundkörpers 1 bzw. 1' und Auflegen auf einen entsprechend geformten Körper, wie beispielsweise einen Fußleisten, eine dauerhafte Formgebung erfolgen.

Auf dieselbe Weise können auch Gegenstände für andere Anwendungszwecke hergestellt werden.

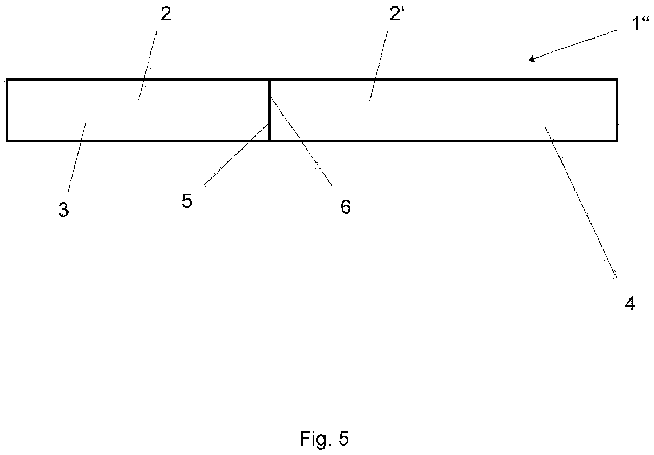

Figur 5 zeigt eine weitere Ausführungsform. Diese umfasst einen Verbundkörper 1", der mehrere Elemente 2, 2' aufweist. Das Element 2 ist durch den ersten Körper 3 und das Element 2' durch den zweiten Körper 4 gebildet. Figur 5 zeigt dabei, dass der erste und der zweite Körper 3, 4 nebeneinander angeordnet sind. Die erste und die zweite Kontaktfläche 5, 6 sind jeweils an Stirnseiten des ersten beziehungsweise zweiten Körpers 3, 4 angeordnet.

Der erste und der zweite Körper 3, 4 sind wie der erste und der zweite Körper 3, 4 des im Zusammenhang mit Figur 1 beschriebenen Verbundkörpers 1 ausgestaltet. Auf die diesbezügliche Beschreibung wird verwiesen.

Figur 5 zeigt dabei, dass der Verbundkörper 1" im dargestellten Ausführungsbeispiel eine Schicht aufweist, in der der erste und zweite Körper 3, 4 nebeneinander angeordnet sind. Auf diese Weise können in unterschiedlichen Bereichen des Verbundkörpers 1" unterschiedliche physikalische Eigenschaften erreicht werden.

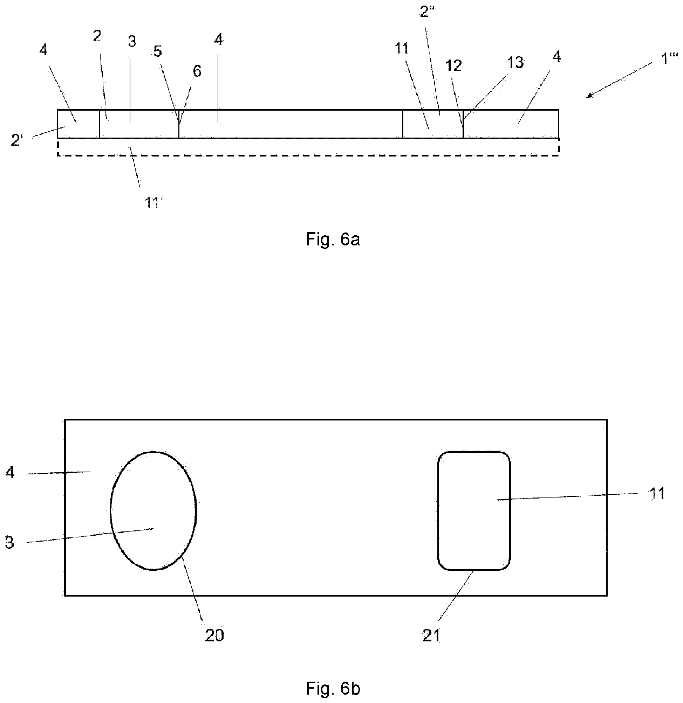

Figuren 6a und 6b zeigen eine weitere Ausführungsform eines Verbundkörpers 1‴. Die Darstellungen zeigen, dass der Verbundkörper 1‴ einen ersten Körper 3, einen zweiten Körper 4 und einen weiteren Körper 11 aufweist. Der zweite Körper 4 weist dabei Ausnehmungen 20, 21 auf, in denen der erste Körper 3 und der weitere Körper 11 angeordnet sind. Auf diese Weise ergibt sich ein Verbundkörper 1‴ mit mehreren Elementen 2, 2', 2", die in einer Schicht des Verbundkörpers 1‴ angeordnet sind.

Figur 6a zeigt durch die gestrichelte Anordnung, dass der Verbundkörper 1‴ darüber hinaus eine weitere Schicht mit einem weiteren Körper 11' aufweisen kann.

Der erste und der zweite Körper 3, 4 sind wiederum an der ersten und zweiten Kontaktfläche 5, 6 miteinander verbunden. Entsprechendes gilt für die dritte und vierte Kontaktfläche 12, 13, an der der zweite Körper 4 mit dem weiteren Körper 11 verbunden ist. Der erste, zweite Körper 3, 4 sowie der weitere Körper 11 weisen darüber hinaus Kontaktflächen auf, mit denen diese an dem (optionalen) weiteren Körper 11' anliegen und mit diesem verbunden sind. Auch diese Verbindung erfolgt in der beschriebenen Weise ohne Klebstoff.

Nachfolgend wird die Herstellung des ersten Körpers 3, des zweiten Körpers 4 und der weiteren Körper 11 bzw. 11' beschrieben. Dabei wird die Herstellung beispielhaft an der Herstellung des ersten Körpers 3 erläutert. Der zweite Körper 4 und der weitere Körper 11 bzw. 11' können grundsätzlich auf dieselbe Weise hergestellt werden. Damit soll die folgende Beschreibung auch entsprechend für die Herstellung des zweiten und des weiteren Körpers 4, 11, 11' gelten.

Der erste Körper 3 wird dadurch hergestellt, dass zunächst ein erstes Basismaterial hergestellt wird. Hierzu werden eine vernetzbare Komponente in unvernetzter Form, ein chemisches Treibmittel und ein Vernetzungsmittel zusammengegeben und gemischt. Hierbei können außerdem ein Füllstoff und Additive, wie z. B. Pigmente, zugegeben werden. Das Mischen kann beispielsweise in einem handelsüblichen Innenmischer erfolgen. Das erste Basismaterial kann dann in eine beispielsweise rechteckige Form gegeben werden, um den ersten Körper 3 herzustellen. Nach dem Einbringen des ersten Basismaterials in die Form werden das chemische Treibmittel wie auch das Vernetzungsmittel durch Zufuhr von Wärme aktiviert. Das kann in einer Heizpresse erfolgen. Dabei expandiert das Basismaterial und bildet eine Poren enthaltende Struktur aus. Das vernetzbare Polymer wird vernetzt. Dies wird in einer Weise durchgeführt, dass nur eine Teilvernetzung des vernetzbaren Polymers erreicht wird. Dadurch entsteht ein elastisch komprimierbarer Körper.

Der zweite Körper 4 und der weitere Körper 11 bzw. 11' können auf dieselbe Weise hergestellt sein. Dabei besteht auch die Möglichkeit, den zweiten Körper 4, und/oder den weiteren Körper 11 bzw. 11' ohne Treibmittel herzustellen. Dann ergibt sich eine nicht expandierte Ausführung des zweiten bzw. weiteren Körpers 4, 11. Hierbei können der zweite Körper 4 und/oder der weitere Körper 11 mit oder ohne Vernetzungsmittel hergestellt sein. Wenn die Herstellung ohne Vernetzungsmittel erfolgt, bleibt die vernetzbare Komponente des zweiten bzw. weiteren Körpers 4, 11 unvernetzt.

Die vernetzbare Komponente umfasst wenigstens eine der in der nachfolgenden Tabelle 1 genannten Verbindungen.

Tabelle 1:

| Vernetzbare Komponente |
| --- |
| Ethylen-Vinylacetat-Copolymer (EVA) |
| funktionalisiertes Ethylen-Butylacrylat-Copolymer |
| funktionalisiertes Ethylen-Vinylacetat-Copolymer |
| Ethylen-Ethylacrylat-Copolymer |
| Ethylen-Methylacrylat-Copolymer |
| Ethylen-(Meth)acrylsäure-Copolymer |
| Ethylen-2-Ethylhexyacrylat-Colpolymer |
| Ethylen-Acrylester-Copolymer |

(fortgesetzt)

| Vernetzbare Komponente |
|---|
| Polyolefin, wie beispielsweise Polyethylen (PE) oder Polypropylen (PP) unter anderem in folgenden Varianten: Polyethylen mit sehr geringer Dichte (VLDPE), Lineares Polyethylen mit geringer Dichte (LLDPE), Polyethylen mit geringer Dichte (LDPE), Polyolefin-Elastomere (z. B. POE) und Ethylen-Propylen-Copolymer (EPM) |
| Styrol-Butadien-Blockcopolymer (SBS) |
| Styrol-Isopren-Blockcopolymer (SIS) |
| Styrol-Isopren-Butadien-Blockcopolymer (SIBS) |
| Styrol-Ethylen-Butadien-Blockcopolymer (SEBS) |
| Styrol-Ethylen-Propylen-Blockcopolymer (SEPS) |
| Hoch-Styrol-Butadien-Copolymer (HSBR, Styrolanteil >50%) |
| Thermoplastisches Polyurethan (TPU) |

Weiterhin kann vorgesehen sein, dass die vernetzbare Komponente zusätzlich ein styrolhaltiges Polymer umfasst. Das styrolhaltige Polymer kann wenigstens eine der in Tabelle 2 genannten Verbindungen aufweisen.

Tabelle 2:

| Styrolhaltiges Polymer |
|---|
| Styrol-Butadien-Blockcopolymer (SBS) |
| Styrol-Isopren-Blockcopolymer (SIS) |
| Styrol-Isopren-Butadien-Blockcopolymer (SIBS) |
| Styrol-Ethylen-Butadien-Blockcopolymer (SEBS) |
| Styrol-Ethylen-Propylen-Blockcopolymer (SEPS) |
| Hoch-Styrol-Butadien-Copolymer (HSBR, vorzugsweise Styrolanteil >50%) |

Außerdem kann vorgesehen sein, dass für das jeweilige Basismaterial ein vernetzbares Elastomer beigegeben wird. Das vernetzbare Elastomer kann insbesondere wenigstens eine der in Tabelle 3 genannten Kautschuk-Verbindungen umfassen.

Tabelle 3:

| Vernetzbares Elastomer |
|---|
| Styrol-Butadien-Kautschuk (SBR) |
| Nitril-Butadien-Kautschuk (NBR) |
| Ethylen-Propylen-Dien-Kautschuk (EPDM) |
| Naturkautschuk (NR) |
| Isopren-Kautschuk (IR) |
| Butadien-Kautschuk (BR) |
| Polyacrylat-Kautschuk (ACM) |
| Polyethylen-Acrylat-Kautschuk (AEM) |

Das Treibmittel dient dazu, das Basismaterial zu expandieren, um einen expandierten und somit Poren aufweisenden Körper zu erhalten. Beispiele für erfindungsgemäß vorgesehene Treibmittel sind insbesondere Azoverbindungen, Hydrazidverbindungen, Nitrosoverbindungen und Carbazidverbindungen.

Insbesondere kann das chemische Treibmittel wenigstens eine der in Tabelle 4 genannten Verbindungen umfassen.

Tabelle 4:

| Treibmittel |
|---|
| Azobisisobutyronitril |
| Azodicarbonamid (ADCA) |
| Di-nitroso-pentamethylentetramin |
| 4,4'-Oxybis(benzolsulfonsäure hydrazid) (OBSH) |
| Azocyclohexylnitril |
| Azodiaminobenzol |
| Benzol-1,3-sulfonylhydrazid |
| Calciumazid |
| 4,4'-Diphenyldisulfonylazid |
| Diphenyl-sulfon-3,3'-disulfohydrazid |
| Benzol-1,3-disulfohydrazid |
| Trihydrazinotriazin |
| p-Toluolsulfonylhydrazid |
| p-Toluolsulfonylsemicarbazid |

Für das jeweilige Basismaterial wird außerdem ein chemisches Vernetzungsmittel zugegeben. Das Vernetzungsmittel kann insbesondere ein Peroxid sein. Insbesondere kann das Vernetzungsmittel wenigstens eine der in Tabelle 5 genannten Verbindungen umfassen.

Tabelle 5:

| Vernetzungsmittel |
|---|
| Cumenhydroperoxid |
| t-Butylperoxid |
| Bis(tert-butylperoxy)-diisopropylbenzol |
| Di(tert-butylperoxyisopropyl)benzol |
| Dicumylperoxid |
| t-Butylperoxybenzoat |
| Di-alkylperoxydicarbonat |
| Diperoxyketale (z. B. 1,1-Di-tert-butylperoxy-3,3,5-trimethylcyclohexan) |
| Ketonperoxide (z. B. Methylethylketon-Peroxide) |
| 4,4- Di-tert-butylperoxy-n-butyl-valerate |
| 3,3,5,7,7-Pentamethyl-1,2,4-trioxepan |
| 2,5-Dimethyl-2,5-di(tert-butylperoxy)hex-3-in |
| Di-tert-butylperoxid |
| 2,5-Dimethyl-2,5-di(tert-butylperoxy)hexan |
| tert-Butylcumylperoxid |
| Di(tert-butylperoxyisopropyl)benzol |
| Dicumylperoxid |
| Butyl-4,4-di(tert-butylperoxi)valerat |
| tert-Butylperoxy-2-ethylhexylcarbonat |
| 1,1-Di-(tert-butylperoxy)-3,3,5-trimethylcyclohexan |

(fortgesetzt)

| Vernetzungsmittel |
|---|
| tert-Butylperoxybenzoat |
| Di-(4-methylbenzoyl)peroxid |
| Dibenzoylperoxid |

Das Vernetzungsmittel wird dabei dem jeweiligen Basismaterial in einer Menge zugegeben, die unter den gegebenen Prozessbedingungen eine Teilvernetzung der vernetzbaren Komponente ermöglicht, jedoch für eine Vollvernetzung zu gering ist.

Dem jeweiligen Basismaterial kann darüber hinaus ein Füllstoff zugegeben werden. Der Füllstoff umfasst vorzugsweise wenigstens eine der in Tabelle 6 genannten Verbindungen.

Tabelle 6:

| Füllstoff |
|---|
| Kieselsäure |
| Kreide |
| Ruß |
| Kieselerde |
| Kaolin |
| Aluminiumhydroxid |
| Natrium-Aluminium-Silikat |
| Glasmehl |
| Holzmehl |
| Nussschalenmehl |
| Lignin |
| Zellulose |

Die in den Tabellen 1 bis 6 genannten Stoffe sind jeweils kommerziell erhältlich.

Nachfolgend werden einzelne Ausführungsbeispiele beschrieben.

Die Tabellen 7, 8 und 9 zeigen Zusammensetzungen, die für das erste Basismaterial des ersten Körpers 3, das zweite Basismaterial des zweiten Körpers 4 beziehungsweise das weitere Basismaterial der weiteren Körper 11, 11'verwendet werden können.

Tabelle 7:

| Rezeptur-Nummer | 1 | 2 | 3 | 4 | 5 | 6 | 7 | 8 | 9 |
|---|---|---|---|---|---|---|---|---|---|
| Ethylen-Vinylacetat-Copolymer | 100 | 100 | 100 | 100 | 100 | 100 | 100 | 100 | 100 |
| Füllstoff (Kieselsäure) | 10 | 10 | 10 | 10 | 10 | 10 | 10 | 10 | 10 |
| Pigmente, Verarbeitungshilfsmittel | 8 | 8 | 8 | 8 | 8 | 8 | 8 | 8 | 8 |
| Treibmittel (Azodicarbonamid) | 5 | 5 | 5 | 5 | 5 | 5 | 5 | 5 | 5 |
| Vemetzungsmittel mit Peroxidgehalt von 40 % | 0,3125 | 0,5 | 0,625 | 0,8125 | 0,9375 | 1,25 | 1,563 | 1,875 | 2,8125 |
| Vemetzungsmittel nur Wirksubstanz | 0,125 | 0,2 | 0,25 | 0,325 | 0,375 | 0,5 | 0,6252 | 0,75 | 1,125 |

(fortgesetzt)

| Rezeptur-Nummer | 1 | 2 | 3 | 4 | 5 | 6 | 7 | 8 | 9 |
|---|---|---|---|---|---|---|---|---|---|
| Schälwiderstand (N/mm) | 2,1 M | 2,2 M | 2,5 M | 2,3 M | 1,4 | 0,9 | 0,2 | 0,2 | keine Klebung |
| Vemetzungsgrad | 25 | 80 | 83 | 86 | 89 | 90 | 93 | 94 | vollvernetzt |

Tabelle 7 zeigt neun verschiedene Rezepturen. Angegeben sind jeweils die Gewichtsanteile bezogen auf die Gesamtmenge an Polymer. So bedeutet beispielsweise die Angabe "100" für Ethylen-Vinylacetat-Copolymer und "10" für Kieselsäure, dass der Mischung 10 Gewichtsanteile (z. B. 10 kg) Kieselsäure auf 100 Gewichtsanteile (z. B. 100 kg) Ethylen-Vinylacetat-Copolymer zugegeben werden. Entsprechendes gilt für die weiteren Bestandteile der Mischung.

Die in Tabelle 7 beispielhaft angegebenen Rezepturen enthalten jeweils Ethylen-Vinylacetat-Copolymer als vernetzbare Komponente. Weiterhin enthalten sind Pigmente und Verarbeitungshilfsmittel sowie Azodicarbonamid als Treibmittel. Verarbeitungshilfsmittel können beispielsweise Zinkcarbonat oder Zinkoxid sein, die die Wirkung des Treibmittels fördern und damit im Herstellungsprozess als Beschleuniger eingesetzt werden können.

Die Rezepturen 1 bis 9 enthalten jeweils dieselbe Menge an Ethylen-Vinylacetat-Copolymer (100 Teile), Füllstoff (10 Teile), Pigmente und Verarbeitungshilfsmittel (8 Teile) und Treibmittel (5 Teile). Die Rezepturen 1 bis 9 enthalten jedoch eine unterschiedliche Menge an Vernetzungsmittel. Die Mengenangabe bezieht sich dabei auf die Wirksubstanz. Aus Gründen der leichteren Verarbeitbarkeit kann das Vernetzungsmittel neben der Wirksubstanz eine Trägersubstanz enthalten. Das in den Rezepturen verwendete Vernetzungsmittel weist jeweils einen Peroxidgehalt von 40 % auf.

Der Gehalt an Vernetzungsmittel variiert bei den Rezepturen 1 bis 9 zwischen 0,125 und 1,125 Gewichtsanteilen pro 100 Gewichtsanteile der Gesamtpolymermenge.

Aus jeder Rezeptur wurden dann einzelne plattenförmige Körper hergestellt. Dies erfolgte dadurch, dass die Komponenten der Rezeptur in einem Innenmischer zu einem Basismaterial gemischt und dann in eine rechteckige Form gegeben werden. Daraufhin werden das Vernetzungsmittel und das Treibmittel aktiviert, um den Körper aus expandiertem Material herzustellen. Dies erfolgte dadurch, dass das Basismaterial in einer Heizpresse mit 170 °C für eine Zeitspanne von 26 Minuten erhitzt wird. Danach wird der Körper aus der Form genommen und abgekühlt.

Tabelle 7 gibt darüber hinaus an, wie stabil die Verbindung zwischen dem ersten und zweiten Körper 3, 4 ist. Bei der Ermittlung der Stabilität der Verbindung wurden zwei identisch ausgebildete Körper derselben Rezeptur wie in Figur 1 dargestellt angeordnet und miteinander verbunden. Die Schichtstärken 7, 8 des ersten und zweiten Körpers 3, 4 betrugen jeweils 4 mm. Die Verbindung erfolgte dadurch, dass die zwei Körper bei einer Temperatur von 130 °C für 4 Minuten aufgewärmt und dann mit der ersten und zweiten Kontaktfläche 5, 6 miteinander in Kontakt gebracht wurden. Sie wurden dann für einen Zeitraum von 16 Minuten miteinander in Kontakt gehalten und dabei abgekühlt. Dies geschieht in einem Tiefziehgerät bei einem Überdruck von 1 bar.

Der für die Verbindung angegebene Wert ist der Schälwiderstand, der nach DIN 1329 ermittelt wurde. Sofern in der Tabelle hinter dem Wert ein "M" angegeben ist, bedeutet dies, dass das Material des ersten oder zweiten Körpers 3, 4 bei dem für den Schälwiderstand angegebenen Wert gerissen ist, ohne dass sich hierbei die Verbindung zwischen dem ersten und dem zweiten Körper 3, 4 gelöst hat.

Die Tabelle gibt weiterhin den Vernetzungsgrad G an. Die Ermittlung des Vernetzungsgrads G erfolgte in der oben beschriebenen Weise analog zu DIN 16892.

Tabelle 7 zeigt deutlich, dass durch die Zugabe der Menge des Vernetzungsmittels gesteuert werden kann, ob in dem fertigen Produkt eine Teilvernetzung oder eine Vollvernetzung erreicht wird. Zudem kann bei gleichbleibenden anderen Prozessparametern, wie z. B. der Temperatur, durch die Menge des Vernetzungsmittels der Grad der Teilvernetzung eingestellt werden.

Rezeptur 9 zeigt dabei ein nicht erfindungsgemäßes Vergleichsbeispiel. Aufgrund der Zugabe des Vernetzungsmittels in einer Menge von 1,125 Gewichtsanteilen pro 100 Gewichtsanteile Polymer wird eine sehr starke Vernetzung erreicht, die auch durch eine weitere Erhöhung der Menge des Vernetzungsmittels nicht mehr gesteigert werden kann. Mit einem solchen vollvernetzten Material kann in der zuvor beschriebenen Weise ohne Klebstoff keine dauerhafte Verbindung zwischen dem ersten und zweiten Körper 3, 4 erreicht werden. Ein Schälwiderstand lässt sich daher nicht ermitteln. Zwei nach der Rezeptur 9 gefertigte Körper können daher nur durch Zugabe eines zusätzlichen Klebstoffs oder einer Klebeschicht miteinander dauerhaft verbunden werden.

Anders ist dies bei den Rezepturen 1 bis 8. Aufgrund der Verwendung des Vernetzungsmittels mit einer im Vergleich zu Rezeptur 9 reduzierten Menge wird eine Teilvernetzung des vernetzbaren Polymers erreicht. Dies ermöglicht es, einen ersten und einen zweiten Körper 3, 4 in der oben beschriebenen Weise miteinander zu verbinden, ohne dass die Zugabe eines Klebstoffs oder einer Klebeschicht erfolgt. Rezepturen 1 bis 8 zeigen dabei einen zunehmenden Vernetzungsgrad, der in der oben beschriebenen Weise ermittelt wurde. Dieser reicht von 25 % bei Rezeptur 1 bis zu 94 % bei Rezeptur 8. Die Rezepturen 7 und 8 mit einem höheren Vernetzungsgrad weisen eine nicht so stark ausgebildete Verbindung auf. Dementsprechend ist der Schälwiderstand mit 0,2 N/mm noch recht gering. Die Beispiele 7 und 8 zeigen jedoch, dass bereits eine nur leichte Absenkung des Vernetzungsgrads im Vergleich zu Rezeptur 9 dazu führt, dass eine Verbindung zwischen den Körpern erreicht werden kann.

Bei den Rezepturen 1 bis 6 wurden im Vergleich zu den Rezepturen 7 und 8 weiter reduzierte Mengen des Vernetzungsmittels verwendet. Dadurch ergibt sich eine Steigerung der Verbindungskraft. Dementsprechend können höhere Werte des Schälwiderstands erreicht werden. Die Beispiele zeigen dabei auch, dass bei einem Vernetzungsgrad von 90 % bereits eine deutlich verbesserte Verbindung mit einem erheblich verbesserten Schälwiderstand erreicht wird. Durch eine Absenkung des Vernetzungsgrads auf Werte von weniger als 90 % kann dieser noch einmal deutlich gesteigert werden. Auch bei einem Vernetzungsgrad von nur 25 % wird eine sehr stabile Verbindung zwischen dem ersten und zweiten Körper 3, 4 mit einem Schälwiderstand von 2,1 N/mm erreicht. Eine zu starke Absenkung des Vernetzungsgrads kann jedoch die Stabilität des Verbundkörpers 1, 1' reduzieren. Daher ist besonders bevorzugt, wenn der Vernetzungsgrad über 50 % liegt. Besonders bevorzugt ist, wenn der Vernetzungsgrad über 80 % liegt.

Tabelle 8:

| Rezeptur-Nummer | 10 | 11 | 12 | 13 |
|---|---|---|---|---|
| LDPE | 75 | 75 | 75 | 75 |
| LLDPE | 25 | 25 | 25 | 25 |
| Füllstoff (Kieselsäure) | 9,5 | 9,5 | 9,5 | 9,5 |
| Pigmente, Verarbeitungshilfsmittel | 5 | 5 | 5 | 5 |
| Treibmittel (Azodicarbonamid) | 2,75 | 2,75 | 2,75 | 2,75 |
| Vernetzungsmittel mit Peroxidgehalt von 40 % | 0,625 | 0,8125 | 0,9375 | 1,25 |
| Vernetzungsmittel nur Wirksubstanz | 0,25 | 0,325 | 0,375 | 0,5 |
| Schälwiderstand (N/mm) | 0,6 M | 0,6 M | 1,5 M | 1,5 M |
| Vernetzungsgrad (%) | 2 | 58 | 70 | 75 |

Tabelle 8 zeigt vier weitere Rezepturen. Diese enthalten jeweils LDPE und LLDPE als vernetzbare Komponente. Enthalten ist weiterhin Kieselsäure als Füllstoff sowie Pigmente und Verarbeitungshilfsmittel sowie Treibmittel. Auch bei Tabelle 8 wurden bei den unterschiedlichen Rezepturen die Mengen des Vernetzungsmittels variiert. Der ermittelte Vernetzungsgrad reicht von 2 % bis 75 %. Für den Schälwiderstand wurden Werte zwischen 0,6 N/mm und 1,5 N/mm ermittelt. Bei allen in Tabelle 8 dargestellten Rezepturen kam es bei der Ermittlung des Schälwiderstands zum Materialbruch. Dies bedeutet, dass die Verbindung zwischen dem ersten und dem zweiten Körper 3, 4 stabiler war als das Material selbst.

Tabelle 9:

| Rezeptur-Nummer | 14 | 15 | 16 | 17 |
|---|---|---|---|---|
| Ethylen-Vinylacetat-Copolymer | 75 | 75 | 87,5 | 75 |
| Hoch-Styrol-Harz-Polymer | 25 | 25 | --- | --- |
| NBR | --- | --- | 12,5 | --- |
| SBS | --- | --- | --- | 25 |
| Kieselsäure | 8 | 8 | 9,5 | 9,5 |
| Pigmente, Verarbeitungshilfsmittel | 2 | 2 | 5 | 5 |
| Treibmittel, z. B. Azodicarbonamid | 2,19 | 1,47 | 2,75 | 2,75 |
| Vernetzungsmittel mit Peroxidgehalt von 40 % | 0,7 | 0,867 | 0,81 | 0,5 |
| Vernetzungsmittel nur Wirksubstanz | 0,28 | 0,3468 | 0,324 | 0,2 |
| Schälwiderstand (N/mm) | 2,6 M | 1,3 M | 1,8 M | 1,2 M |
| Vernetzungsgrad (%) | 91 | 85 | 85 | 82 |

Tabelle 9 zeigt vier weitere Rezepturen. Diese enthalten als vernetzbare Komponente Ethylen-Vinylacetat-Copolymer. Die Rezepturen 14 und 15 enthalten dabei zusätzlich Hoch-Styrol-Harz-Polymer. In Rezepturen 16 und 17 ist jeweils ein vernetzbares Elastomer enthalten. Rezeptur 16 enthält insoweit NBR und Rezeptur 17 SBS. Der ermittelte Vernetzungsgrad G liegt zwischen 82 % und 91 %.

Bei allen vier Rezepturen aus Tabelle 9 wurde eine gute Verbindung zwischen dem ersten und zweiten Körper 3, 4 erreicht. Der ermittelte Schälwiderstand reicht von 1,2 N/mm bis 2,6 N/mm. Dabei kam es bei der Ermittlung des Schälwiderstands in allen Fällen zum Materialbruch. Dies bedeutet, dass die Verbindung eine höhere Festigkeit aufweist als das Material selbst.

## Patentansprüche

1. Verfahren zur Herstellung eines Gegenstandes, insbesondere eines orthopädischen Gegenstands (10, 10') , mit einem mehrere Elemente (2, 2', 2") aufweisenden elastisch komprimierbaren Verbundkörper (1, 1'), umfassend:

   - Bereitstellen eines ersten plattenförmig ausgebildeten Körpers (3) aus einem ersten Material, wobei das erste Material expandiert ist, wobei der erste Körper (3) eine erste Kontaktfläche (5) aufweist,
   - Bereitstellen eines zweiten plattenförmig ausgebildeten Körpers (4) aus einem zweiten Material, wobei der zweite Körper (6) eine zweite Kontaktfläche aufweist,
   wobei das erste und das zweite Material jeweils eine vernetzbare Komponente aufweisen, die ein Polymer umfasst, das vernetzbar ist, wobei das vernetzbare Polymer des ersten Körpers (3) in teilvernetzter Form vorliegt, wobei das vernetzbare Polymer des zweiten Körpers (4) in teilvernetzter Form vorliegt, wobei der erste Körper (3) dadurch hergestellt ist, dass zunächst ein erstes Basismaterial hergestellt wird, indem die vernetzbare Komponente in unvernetzter Form, ein chemisches Treibmittel und ein Vernetzungsmittel zusammen gemischt werden, dass das chemische Treibmittel und das Vernetzungsmittel dann aktiviert werden, sodass das Basismaterial expandiert und das vernetzbare Polymer teilvernetzt wird, um das expandierte Material des ersten Körpers mit teilvernetzten Polymeren zu erhalten, wobei der erste Körper (3) weicher als der zweite Körper (4) ist, wobei der erste Körper eine Härte zwischen 5 und 50 Shore A aufweist, wobei der zweite Körper eine Härte zwischen 20 und 75 Shore A aufweist, und
   - Herstellen des Verbundkörpers (1, 1') mit einer dauerhaften Verbindung zwischen dem ersten und dem zweiten Körper (3, 4), dadurch, dass der erste Körper (3) und der zweite Körper (4) erwärmt und die erste Kontaktfläche (5) des ersten Körpers (3) mit der zweiten Kontaktfläche (6) des zweiten Körpers (4) in Kontakt gebracht und für eine Zeitspanne in Kontakt gehalten werden.

2. Verfahren nach Anspruch 1, **dadurch gekennzeichnet, dass** die vernetzbare Komponente des ersten und/oder des zweiten Materials wenigstens eine Verbindung ausgewählt aus einer Gruppe bestehend aus einem peroxidisch vernetzbaren thermoplastischen Polymer und einem peroxidisch vernetzbaren thermoplastischen Elastomer umfasst.

3. Verfahren nach Anspruch 2, **dadurch gekennzeichnet, dass** die vernetzbare Komponente des ersten und/oder des zweiten Materials wenigstens eine Verbindung ausgewählt aus einer Gruppe bestehend aus Ethylen-Butylacrylat-Copolymer (EBA), Ethylen-Vinylacetat-Copolymer (EVA), funktionalisiertes Ethylen-Butylacrylat-Copolymer, funktionalisiertes Ethylen-Vinylacetat-Copolymer, Ethylen-Ethylacrylat-Copolymer, Ethylen-Methylacrylat-Copolymer, Ethylen-(Meth)acrylsäure-Copolymer, Ethylen-2-Ethylhexyacrylat-Colpolymer, Ethylen-Acrylester-Copolymer, Polyolefin, Styrol-Butadien-Blockcopolymer (SBS), Styrol-Isopren-Blockcopolymer (SIS), Styrol-Isopren-Butadien-Blockcopolymer (SIBS), Styrol-Ethylen-Butadien-Blockcopolymer (SEBS), Styrol-Ethylen-Propylen-Blockcopolymer (SEPS), Hoch-Styrol-Butadien-Copolymer (HSBR) und Thermoplastisches Polyurethan (TPU) umfasst.

4. Verfahren nach einem der Ansprüche 1 bis 3, **dadurch gekennzeichnet, dass** die vernetzbare Komponente ein styrolhaltiges Polymer umfasst.

5. Verfahren nach einem der Ansprüche 1 bis 4, **dadurch gekennzeichnet, dass** wenigstens eines des ersten und des zweiten Materials zusätzlich wenigstens ein vernetzbares Elastomer aufweisen, das in dem ersten bzw. zweiten expandierten Material in teilvernetzter Form vorliegt.

6. Verfahren nach einem der Ansprüche 1 bis 5, **dadurch gekennzeichnet, dass** das erste und/oder das zweite Material einen Füllstoff aufweist.

7. Verfahren nach einem der Ansprüche 1 bis 6, **dadurch gekennzeichnet, dass** der zweite Körper (4) dadurch hergestellt ist, dass zunächst ein zweites Basismaterial hergestellt wird, indem die vernetzbare Komponente in unvernetzter Form, ein chemisches Treibmittel und ein Vernetzungsmittel zusammen gemischt werden, dass das chemische Treibmittel und das Vernetzungsmittel dann aktiviert werden, sodass das zweite Basismaterial expandiert und das vernetzbare Polymer teilvernetzt wird, um das expandierte zweite Material des zweiten Körpers mit teilvernetzten Polymeren zu erhalten.

8. Verfahren nach einem der Ansprüche 1 bis 7, **dadurch gekennzeichnet, dass** das Vernetzungsmittel in dem ersten Basismaterial zur Erreichung der angestrebten Teilvernetzung vorzugsweise in einer Menge von 0,05 bis 0,5 Gew.-%, bestimmt als Aktivsubstanzgehalt an Peroxid bezogen auf die Masse des ersten bzw. zweiten Basismaterials, enthalten ist.

9. Verfahren nach einem der Ansprüche 1 bis 8, **dadurch gekennzeichnet, dass** das Vernetzungsmittel ein Peroxid umfasst.

10. Verfahren nach einem der Ansprüche 1 bis 9, **dadurch gekennzeichnet, dass** der Vernetzungsgrad des ersten Körpers und/oder des zweiten Körpers unter 92 % liegt.

11. Verfahren nach einem der Ansprüche 1 bis 10, **dadurch gekennzeichnet, dass** der Vernetzungsgrad des ersten Körpers (3) und/oder des zweiten Körpers (4) über 25 % liegt.

12. Verfahren nach einem der Ansprüche 1 bis 11, **dadurch gekennzeichnet, dass** der erste und der zweite Körper (3, 4) unterschiedliche physikalische Eigenschaften aufweisen.

13. Verfahren nach Anspruch 12, **dadurch gekennzeichnet, dass** die Dichte des ersten und des zweiten Körpers (3, 4) zwischen 0,05 g/cm$^3$ und 0,7 g/cm$^3$ beträgt.

14. Kombination von Gegenständen, umfassend

   - einen ersten plattenförmigen Körper (3) aus einem ersten Material, das expandiert ist und das eine vernetzbare Komponente umfasst, wobei die vernetzbare Komponente ein vernetzbares Polymer umfasst, das in dem ersten Körper (3) in teilvernetzter Form vorliegt, wobei der erste Körper (3) eine erste Kontaktfläche (5) aufweist, und
   - einen zweiten plattenförmigen Körper (4) aus einem zweiten Material, das eine vernetzbare Komponente umfasst, wobei die vernetzbare Komponente ein vernetzbares Polymer umfasst, das in dem zweiten Körper (4) in unvernetzter oder in teilvernetzter Form vorliegt, wobei der zweite Körper (4) eine zweite Kontaktfläche (6) aufweist,

   wobei der erste und der zweite Körper (3, 4) an der ersten und der zweiten Kontaktfläche (5, 6) miteinander verbindbar oder verbunden sind, wobei der erste Körper (3) dadurch hergestellt ist, dass zunächst ein erstes Basismaterial hergestellt wird, indem die vernetzbare Komponente in unvernetzter Form, ein chemisches Treibmittel und ein Vernetzungsmittel zusammen gemischt werden, dass das chemische Treibmittel und das Vernetzungsmittel dann aktiviert werden, sodass das Basismaterial expandiert und das vernetzbare Polymer teilvernetzt wird, um das expandierte Material des ersten Körpers mit teilvernetzten Polymeren zu erhalten, wobei der erste Körper weicher als der zweite Körper ist, wobei der erste Körper eine Härte zwischen 5 und 50 Shore A aufweist, wobei der zweite Körper eine Härte zwischen 20 und 75 Shore A aufweist.

15. Schuhbestandteil, umfassend eine Kombination von Gegenständen nach Anspruch 14, wobei der erste Körper (3) mit der ersten Kontaktfläche (5) an der zweiten Kontaktfläche (6) des zweiten Körpers (4) anliegt, wobei der erste Körper (3) und der zweite Körper (4) dauerhaft verbunden sind.

**Claims**

1. Method for manufacturing an object, in particular an orthopaedic object (10, 10'), with an elastically compressible composite body (1, 1') having a plurality of elements (2, 2', 2"), comprising:

   - providing a first plate-shaped body (3) of a first material, wherein the first material is expanded, wherein the first body (3) has a first contact face (5),

- providing a second plate-shaped body (4) of a second material, wherein the second body (6) has a second contact face,

wherein the first and the second material each include a crosslinkable component comprising a crosslinkable polymer, wherein the crosslinkable polymer of the first body (3) is in a partially crosslinked form, wherein the crosslinkable polymer of the second body (4) is in a partially crosslinked form, wherein the first body (3) is manufactured in that initially a first base material is manufactured, by mixing together the crosslinkable component in uncrosslinked form, a chemical blowing agent and a crosslinking agent, in that subsequently the chemical blowing agent and the crosslinking agent are activated in such a way that the base material expands and the crosslinkable polymer becomes partially crosslinked, so as to obtain the expanded material of the first body with partially crosslinked polymers, wherein the first body (3) is softer than the second body (4), wherein the first body has a hardness between 5 and 50 Shore A, wherein the second body has a hardness between 20 and 75 Shore A,

and

- manufacturing the composite body (1, 1') with a durable connection between the first and the second body (3, 4), in that the first body (3) and the second body (4) are heated and the first contact face (5) of the first body (3) is brought into contact with the second contact face (6) of the second body (4) and held in contact for a period of time.

2. Method according to claim 1, **characterised in that** the crosslinkable component of the first and/or of the second material comprises at least one compound selected from a group consisting of a peroxide-crosslinkable thermoplastic polymer and a peroxide-crosslinkable thermoplastic elastomer.

3. Method according to claim 2, **characterised in that** the crosslinkable component of the first and/or of the second material comprises at least one compound selected from a group consisting of ethylene butyl acrylate copolymer (EBA), ethylene vinyl acetate copolymer (EVA), functionalised ethylene butyl acrylate copolymer, functionalised ethylene vinyl acetate copolymer, ethylene ethyl acrylate copolymer, ethylene methyl acrylate copolymer, ethylene (meth)acrylic acid copolymer, ethylene 2-ethylhexyl acrylate copolymer, ethylene acryl ester copolymer, polyolefin, styrene butadiene block copolymer (SBS), styrene isoprene block copolymer (SIS), styrene isoprene butadiene block copolymer (SIBS), styrene ethylene butadiene block copolymer (SEBS), styrene ethylene propylene block copolymer (SEPS), high styrene butadiene copolymer (HSBR) and thermoplastic polyurethane (TPU).

4. Method according to any of claims 1 to 3, **characterised in that** the crosslinkable component comprises a styrene-containing polymer.

5. Method according to any of claims 1 to 4, **characterised in that** at least one of the first and the second material additionally includes at least one crosslinkable elastomer, which is present in the first and/or second expanded material in a partially crosslinked form.

6. Method according to any of claims 1 to 5, **characterised in that** the first and/or the second material includes a filler.

7. Method according to any of claims 1 to 6, **characterised in that** the second body (4) is manufactured **in that** initially a second base material is manufactured, by mixing together the crosslinkable component in uncrosslinked form, a chemical blowing agent and a crosslinking agent, **in that** subsequently the chemical blowing agent and the crosslinking agent are activated in such a way that the second base material expands and the crosslinkable polymer becomes partially crosslinked, so as to obtain the expanded second material of the second body with partially crosslinked polymers.

8. Method according to any of claims 1 to 7, **characterised in that**, to obtain the desired partial crosslinking, the crosslinking agent in the first base material is preferably contained in an amount of 0.05 to 0.5 % by weight, determined as a peroxide active substance content based on the mass of the first and/or second base material.

9. Method according to any of claims 1 to 8, **characterised in that** the crosslinking agent comprises a peroxide.

10. Method according to any of claims 1 to 9, **characterised in that** the degree of crosslinking of the first body and/or of the second body is below 92%.

11. Method according to any of claims 1 to 10, **characterised in that** the degree of crosslinking of the first body (3) and/or of the second body (4) is over 25%.

**12.** Method according to any of claims 1 to 11, **characterised in that** the first and the second body (3, 4) have different physical properties.

**13.** Method according to claim 12, **characterised in that** the density of the first and of the second body (3, 4) is between 0.05 g/cm$^3$ and 0.7 g/cm$^3$.

**14.** Combination of objects, comprising

- a first plate-shaped body (3) of a first material which is expanded and comprises a crosslinkable component, wherein the crosslinkable component comprises a crosslinkable polymer which is present in the first body (3) in a partially crosslinked form, wherein the first body (3) has a first contact face (5), and
- a second plate-shaped body (4) of a second material which comprises a crosslinkable component, wherein the crosslinkable component comprises a crosslinkable polymer which is present in the second body (4) in an uncrosslinked or partially crosslinked form, wherein the second body (4) has a second contact face (6),

wherein the first and the second body (3, 4) are interconnectable or interconnected at the first and the second contact face (5, 6), wherein the first body (3) is manufactured in that initially a first base material is manufactured, by mixing together the crosslinkable component in uncrosslinked form, a chemical blowing agent and a crosslinking agent, in that subsequently the chemical blowing agent and the crosslinking agent are activated in such a way that the base material expands and the crosslinkable polymer becomes partially crosslinked, so as to obtain the expanded material of the first body with partially crosslinked polymers, wherein the first body is softer than the second body, wherein the first body has a hardness between 5 and 50 Shore A, wherein the second body has a hardness between 20 and 75 Shore A.

**15.** Shoe component, comprising a combination of objects according to claim 14, wherein the first contact face (5) of the first body (3) is positioned against the second contact face (6) of the second body (4), wherein the first body (3) and the second body (4) are durably connected.

**Revendications**

**1.** Procédé de fabrication d'un objet, en particulier un objet orthopédique (10, 10'), doté d'un corps composite (1, 1') pouvant être comprimé élastiquement présentant une pluralité d'éléments (2, 2', 2"), comprenant :

- obtention d'un premier corps (3) réalisé en forme de plaque en un premier matériau, dans lequel le premier matériau est expansé, dans lequel le premier corps (3) présente une première surface de contact (5),
- obtention d'un second corps (4) réalisé en forme de plaque en un second matériau, dans lequel le second corps (6) présente une seconde surface de contact,
dans lequel le premier et le second matériau présentent chacun un composant réticulable, qui comprend un polymère qui est réticulable, dans lequel le polymère réticulable du premier corps (3) se présente sous une forme partiellement réticulée, dans lequel le polymère réticulable du second corps (4) se présente sous une forme partiellement réticulée, dans lequel le premier corps (3) est fabriqué par le fait qu'un premier matériau de base est d'abord fabriqué en mélangeant ensemble le composant réticulable sous une forme non réticulée, un agent porogène chimique et un agent de réticulation, que l'agent porogène chimique et l'agent de réticulation sont ensuite activés, de telle sorte que le matériau de base subit une expansion et le polymère réticulable est partiellement réticulé pour obtenir le matériau expansé du premier corps avec des polymères partiellement réticulés, dans lequel le premier corps (3) est plus mou que le second corps (4), dans lequel le premier corps présente une dureté comprise entre 5 et 50 Shore A, dans lequel le second corps présente une dureté comprise entre 20 et 75 Shore A,
et
- fabrication du corps composite (1, 1') avec une liaison permanente entre le premier et le second corps (3, 4) par le fait que le premier corps (3) et le second corps (4) sont chauffés et la première surface de contact (5) du premier corps (3) est amenée en contact avec la seconde surface de contact (6) du second corps (4) et elles sont maintenues en contact pendant un laps de temps.

**2.** Procédé selon la revendication 1, **caractérisé en ce que** le composant réticulable du premier et/ou du second matériau comprend au moins un composé sélectionné dans un groupe constitué d'un polymère thermoplastique réticulable sous action d'un peroxyde et d'un élastomère thermoplastique réticulable sous action d'un peroxyde.

**3.** Procédé selon la revendication 2, **caractérisé en ce que** le composant réticulable du premier et/ou du second matériau comprend au moins un composé sélectionné dans un groupe constitué de copolymère éthylène-acrylate de butyle (EBA), copolymère éthylène-acétate de vinyle (EVA), copolymère éthylène-acrylate de butyle fonctionnalisé, copolymère éthylène-acétate de vinyle fonctionnalisé, copolymère éthylène-acrylate d'éthyle, copolymère éthylène-acrylate de méthyle, copolymère éthylène-acide (méth)acrylique, copolymère éthylène-acrylate de 2-éthylhexyle, copolymère éthylène-ester acrylique, polyoléfine, copolymère séquencé styrène-butadiène (SBS), copolymère séquencé styrène-isoprène (SIS), copolymère séquencé styrène-isoprène-butadiène (SIBS), copolymère séquencé styrène-éthylène-butadiène (SEBS), copolymère séquencé styrène-éthylène-propylène (SEPS), copolymère styrène-butadiène à haute teneur en styrène (HSBR) et polyuréthane thermoplastique (TPU).

**4.** Procédé selon l'une des revendications 1 à 3, **caractérisé en ce que** le composant réticulable comprend un polymère contenant du styrène.

**5.** Procédé selon l'une des revendications 1 à 4, **caractérisé en ce qu'**au moins un parmi le premier et le second matériau présente en plus au moins un élastomère réticulable qui se présente sous une forme partiellement réticulée dans respectivement le premier et le second matériau expansé.

**6.** Procédé selon l'une des revendications 1 à 5, **caractérisé en ce que** le premier et/ou le second matériau présentent une matière de charge.

**7.** Procédé selon l'une des revendications 1 à 6, **caractérisé en ce que** le second corps (4) est fabriqué par le fait qu'un second matériau de base est d'abord fabriqué en mélangeant ensemble le composant réticulable sous une forme non réticulée, un agent porogène chimique et un agent de réticulation, que l'agent porogène chimique et l'agent de réticulation sont ensuite activés, de telle sorte que le second matériau de base subit une expansion et le polymère réticulable est partiellement réticulé pour obtenir le second matériau expansé du second corps avec des polymères partiellement réticulés.

**8.** Procédé selon l'une des revendications 1 à 7, **caractérisé en ce que**, pour obtenir la réticulation partielle souhaitée, l'agent de réticulation dans le premier matériau de base est contenu de préférence en une quantité de 0,05 à 0,5 % en poids, déterminée comme teneur en substance active peroxyde par rapport à la masse respectivement du premier et du second matériau de base.

**9.** Procédé selon l'une des revendications 1 à 8, **caractérisé en ce que** l'agent de réticulation comprend un peroxyde.

**10.** Procédé selon l'une des revendications 1 à 9, **caractérisé en ce que** le degré de réticulation du premier corps et/ou du second corps est inférieur à 92 %.

**11.** Procédé selon l'une des revendications 1 à 10, **caractérisé en ce que** le degré de réticulation du premier corps (3) et/ou du second corps (4) est supérieur à 25 %.

**12.** Procédé selon l'une des revendications 1 à 11, **caractérisé en ce que** le premier et le second corps (3, 4) présentent des propriétés physiques différentes.

**13.** Procédé selon la revendication 12, **caractérisé en ce que** la densité du premier et du second corps (3, 4) est comprise entre 0,05 g/cm$^3$ et 0,7 g/cm$^3$.

**14.** Combinaison d'objets, comprenant

- un premier corps (3) en forme de plaque en un premier matériau qui est expansé et qui comprend un composant réticulable, dans lequel le composant réticulable comprend un polymère réticulable qui se présente dans le premier corps (3) sous une forme partiellement réticulée, dans lequel le premier corps (3) présente une première surface de contact (5), et
- un second corps (4) en forme de plaque en un second matériau qui comprend un composant réticulable, dans lequel le composant réticulable comprend un polymère réticulable qui se présente dans le second corps (4) sous une forme non réticulée ou partiellement réticulée, dans lequel le second corps (4) présente une seconde surface de contact (6),

dans lequel le premier et le second corps (3, 4) peuvent être ou sont reliés l'un à l'autre sur la première et la seconde surface de contact (5, 6), dans lequel le premier corps (3) est fabriqué par le fait qu'un premier matériau de base est d'abord fabriqué en mélangeant ensemble le composant réticulable sous la forme non réticulée, un agent porogène chimique et un agent de réticulation, que l'agent porogène chimique et l'agent de réticulation sont ensuite activés, de telle sorte que le matériau de base subit une expansion et le polymère réticulable est partiellement réticulé pour obtenir le matériau expansé du premier corps avec des polymères partiellement réticulés, dans lequel le premier corps est plus mou que le second corps, dans lequel le premier corps présente une dureté comprise entre 5 et 50 Shore A, dans lequel le second corps présente une dureté comprise entre 20 et 75 Shore A.

15. Élément de chaussure, comprenant une combinaison d'objets selon la revendication 14, dans lequel le premier corps (3) repose avec la première surface de contact (5) contre la seconde surface de contact (6) du second corps (4), dans lequel le premier corps (3) et le second corps (4) sont reliés de manière permanente.

Fig. 1

Fig. 2

Fig. 3

Fig. 4

...

Fig. 5

Fig. 6a

Fig. 6b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102004014609 A1

- EP 3267817 A1